(19) Europäisches Patentamt
European Patent Office
Office européen des brevets

(11) Veröffentlichungsnummer: **0 374 751 A2**

(12) # EUROPÄISCHE PATENTANMELDUNG

(21) Anmeldenummer: **89123224.1**

(22) Anmeldetag: **15.12.89**

(51) Int. Cl.5: **B41M 7/02, B41M 1/36, G03C 1/815**

Patentansprüche für folgenden Vertragsstaat: ES.

(30) Priorität: **19.12.88 CH 4674/88**

(43) Veröffentlichungstag der Anmeldung: **27.06.90 Patentblatt 90/26**

(84) Benannte Vertragsstaaten: **BE DE ES FR GB IT NL**

(71) Anmelder: **CIBA-GEIGY AG**
**Klybeckstrasse 141**
**CH-4002 Basel(CH)**

(72) Erfinder: **Vieira, Eric, Dr.**
**rue d'Or 5**
**CH-1700 Fribourg(CH)**
Erfinder: **Laver, Hugh Stephen, Dr.**
**Route de Schiffenen 12**
**CH-1700 Fribourg(CH)**

(74) Vertreter: **Zumstein, Fritz, Dr. et al**
**Bräuhausstrasse 4**
**D-8000 München 2(DE)**

(54) **Wasserlösliche Verbindungen als Lichtschutzmittel.**

(57) Verbindungen der Formel I
$U\{SOL)_{n'}$ (I),
worin n′ eine Zahl von 1 bis 4, U einen Rest eines UV-Absorbers vom Typ Hydroxyphenyl-benztriazol, Zimtsäure oder Triazin und SOL eine Gruppe der Formel Ia

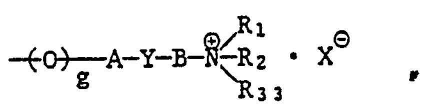

(Ia)

bedeuten. Die Symbole in der Formel Ia haben die in Anspruch 1 angegebene Bedeutung. Die Verbindungen, die zum Teil neu sind, eignen sich als Lichtschutzmittel, insbesondere für Aufzeichnungsmaterialien und Tinten für den Tintenstrahldruck.

EP 0 374 751 A2

## Wasserlösliche Verbindungen als Lichtschutzmittel

Die vorliegende Erfindung betrifft die Lichtstabilisierung von Aufzeichnungsmaterialien für den Tintenstrahldruck durch Zusatz von wasserlöslichen Stabilisatoren sowie neue Verbindungen und deren Verwendung als Stabilisatoren.

Das Drucken mittels Tintenstrahl ist ein Druckverfahren, das durch elektrische Signale gesteuert werden kann. Dabei wird ein feiner Strahl von Tintentröpfchen durch eine Düse auf das Aufzeichnungsmaterial gespritzt. Die Tinte ist eine Lösung eines Farbstoffes in einem wässrigen oder nicht-wässrigen Lösungsmittel. Das Aufzeichnungsmaterial soll den Farbstoff der Tinte rasch und dauerhaft aufnehmen. Meist verwendet man hierfür besonders präparierte Papiere oder Plastikfolien, die mit einer farbstoffbindenden Schicht versehen sind. Wegen der Feinheit der Düsen werden Pigmente kaum verwendet, sondern vorwiegend Farbstoffe, die im Medium des Tintenstrahls vollständig gelöst sind. Diese Farbstoffe haben allerdings generell eine geringere Lichtechtheit als die in konventionellen Druckfarben üblichen Farb-Pigmente. Als Folge davon sind im Tintenstrahldruck hergestellte Aufzeichnungen unter Lichtzutritt nur beschränkt lagerfähig. Bei längerer Lagerung am Licht beginnen sie zu verblassen oder sich zu verfärben. Um dieses Problem zu lösen, wurde beispielsweise in US-A-4,256,493 vorgeschlagen, der Tinte einen wasserlöslichen UV-Absorber zuzusetzen. Hierfür wurden anionische UV-Absorber der Benzophenon-Klasse, die Sulphonatgruppen enthalten, eingesetzt, um Verträglichkeit mit den meistens anionischen Farbstoffen der Tinte zu gewährleisten. Solche Benzophenonderivate und deren Salze besitzen jedoch den Nachteil, dass sie mit bestimmten Farbstoffen, vor allem schwarzen Farbstoffen, Verfärbungen verursachen. Weiterhin kann die Zugabe solcher Additive eine Herabsetzung der Wasserbeständigkeit des Druckes ver ursachen, da die anionischen UV-Absorber mit dem ebenfalls anionischen Farbstoff für die kationischen Beizmittel im Aufzeichnungsmaterial konkurrenzieren.

Ein weiteres Problem beim Tintenstrahldruck ist die Beständigkeit des bedruckten Aufzeichnungsmaterials gegenüber Wasser, die mit der Lichtechtheit zusammenhängt. Wenn keine oder nur ungenügende Gegenmassnahmen getroffen werden, lösen sich die Farbstoffe beim Kontakt mit Wasser aus dem Aufzeichnungsmaterial und verlaufen. Weiterhin kann bei der Lagerung des bedruckten Aufzeichnungsmaterials eine Diffusion der Farbstoffe infolge Feuchtigkeit stattfinden. Das hat eine Minderung der Bildqualität zur Folge.

Es ist ferner wichtig, dass die Tinten, welche in rascher Folge auf das Aufzeichnungsmaterial gespritzt werden, wie das beispielsweise beim 4-Farbentintenstrahldruck der Fall ist, nicht ineinander verlaufen und ein unscharfes Bild verursachen.

Um das Problem der Wasserfestigkeit zu lösen, wird in der DE-A-3 640 359 vorgeschlagen, eine Tintenempfangsschicht bereitzustellen, welche ein wasserlösliches Polymer mit einem Gehalt an 2 bis 30 Gew.% eines kationischen Harzes enthält. Die kationischen Ladungen fixieren die meist anionischen Farbstoffe der Tinte in der Tintenaufnahmeschicht. In den US-PS-4 659 382, 4 664 708, 4 680 332 und 4 705 567 hat man bereits vorgeschlagen, die anionischen Farbstoffe mit einem kationischen Polymer reagieren zu lassen. Das unlösliche Produkt wird dann in dem Tintenmedium dispergiert, um den daraus resultierenden Tintenstrahldrucken gute Wasserbeständigkeit zu verleihen. Diese ungelöste, Polymergebundene Farbstoffe erhöhen allerdings die Neigung zur Verstopfung der Tintendüsen.

Aus der US-PS-4 685 968 und den EP-A-0 224 909 und 0 262 861 ist es bekannt, dass das Austauschen der $Na^+$-Ionen eines anionischen Farbstoffes und das Ersetzen durch quaternäre Amine, wie z.B. Ammonium-, quaternäre Alkanolamin- und Amidoamin-Ionen, eine Tinte mit verbesserter Wasserbeständigkeit und verringerter Tendenz zur Düsenverstopfung ergibt.

Alle bisherigen Vorschläge zur Bereitstellung eines wasserfesten Aufzeichnungsmaterials für Tintenstrahldruck konnten nur auf Kosten der Lichtechtheit erreicht werden (vgl. G.F. Bradley and H.S. Laver: Fastness of Ink-Jet Prints from Water-based inks, The Soc. of Electrophotography of Japan, Japan, pp. 167-169) 1988.

Es wurde nun gefunden, dass bestimmte Stabilisatoren, welche mindestens eine quaternäre Ammoniumgruppe enthalten, wirksame Lichtschutzmittel sind und sich insbesondere für die Stabilisierung von Aufzeichnungsmaterialien und Tinten, insbesondere für den Tintenstrahldruck, eignen.

Kationische UV-Absorber, welche mindestens eine quaternäre Ammoniumgruppe enthalten, sind bekannt. So beschreiben beispielsweise JP-A-61-192 781 und 50-121 178 kationische UV-Absorber vom Benztriazol-Typ, JP-A-61-192 778 vom Benzophenon-Typ und JP-A-61-192 780 vom 2-Cyanozimtsäure-Typ. Sie werden zum Lichtschutz von Ueberzügen, Lacken, Farbstoffen, Kosmetika und in kationischen Galvanolackierungen verwendet.

Gegenstand vorliegender Erfindung ist ein Aufzeichnungsmaterial enthaltend als Stabilisator mindestens

eine Verbindung der Formel I

U{SOL)$_{n'}$ (I),

worin n' eine Zahl von 1 bis 4 bedeutet,

U ein Rest eines UV-Absorbers vom Typ Hydroxyphenyl-benztriazol, Zimtsäure oder Hydroxyphenyl-Triazin ist,

SOL, gleich oder verschieden, eine Gruppe der Formel Ia

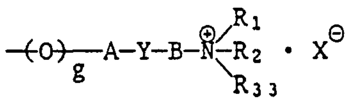
(Ia)

bedeutet, worin

g für 0 oder 1 steht,

A eine direkte Bindung, $C_1$-$C_6$-Alkylen oder -Alkyliden oder eine Gruppe der Formel Ib

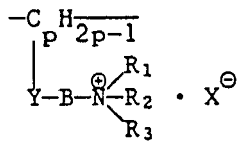
(Ib)

bedeutet, wobei p eine Zahl von 1 bis 6 ist,

Y eine direkte Bindung oder eine der folgenden Gruppen ist:

-CO-, -COO-, -OOC-, -CO-N(R')-, -(R')N-CO-, $-SO_2-N(R')-$, $-(R')N-SO_2-$,

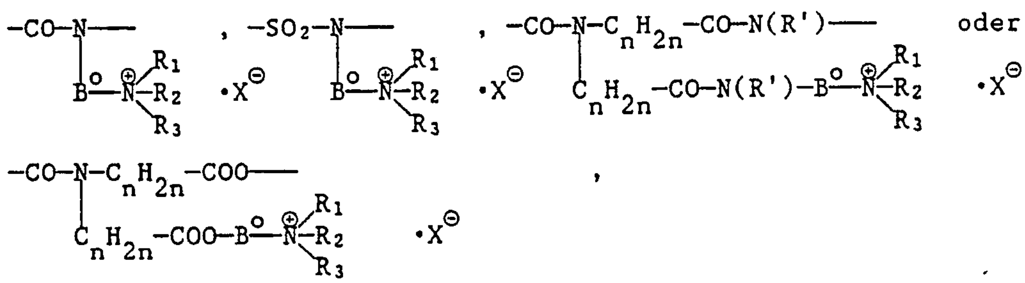
, , oder ,

worin n eine Zahl von 1 bis 4 bedeutet, B eine direkte Bindung bedeutet oder ein gegebenenfalls mit OH substituiertes $C_2$-$C_6$-Alkylen bzw. -Alkyliden ist, welches gegebenenfalls durch ein -O- oder durch ein oder zwei - $\overset{\oplus}{N}X^{\ominus}(R')_2$- unterbrochen ist, oder eine Gruppe der Formeln Ic oder IC

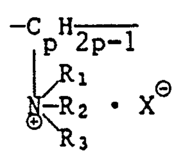
(Ic) oder

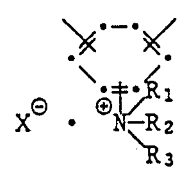
(IC)

ist, oder die Gruppe

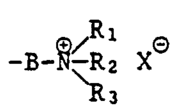

für einen gesättigten oder ungesättigten 1 bis 3-kernigen N-Heterocyklus steht, der 1-4 N-Atome als Ringglieder enthält, von denen mindestens eines quaterniert ist, unter den Bedingungen, dass

a) im Falle g = 1, A, Y und B nicht gleichzeitig eine direkte Bindung bedeuten;

b) im Falle g = 1 und A = direkte Bindung, Y -CO- oder eine direkte Bindung bedeutet; und

c) im Falle B = direkte Bindung, auch Y eine direkte Bindung ist; R′ Wasserstoff, $C_1$-$C_4$-Alkyl oder $C_2$-$C_3$-Hydroxyalkyl ist,

$B^o$-$(CH_2)_m$ oder eine der folgenden Gruppen bedeutet:

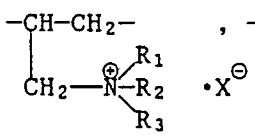
,
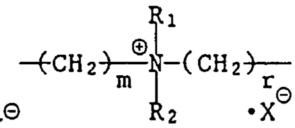
oder
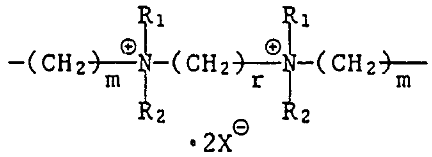

wobei m und r unabhängig voneinander 2 oder 3 bedeuten,

$R_{33}$ eine Bedeutung von $R_3$ hat oder für eine Gruppe der Formeln Id und Ie

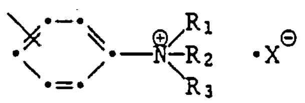
(Id)

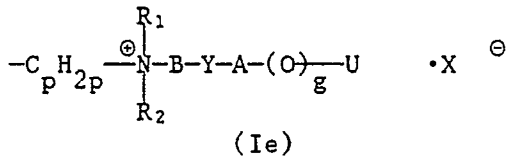
(Ie)

steht, worin g, p, B, Y, A und U die zuvor genannte Bedeutung haben,

$R_1$, $R_2$ und $R_3$ unabhängig voneinander Wasserstoff, $C_1$-$C_8$-Alkyl oder durch ein -COOR″ oder 1 bis 3 OH substituiertes $C_1$-$C_8$-Alkyl, durch ein oder mehrere -O- unterbrochenes $C_2$-$C_8$-Hydroxyalkyl, mit einem OH substituiertes -($C_1$-$C_8$)Alkylen-$COO^{\ominus}$, -($C_1$-$C_8$)Alkyliden-$COO^{\ominus}$, -($C_2$-$C_8$)Alkylen-$SO_3^{\ominus}$ oder -($C_2$-$C_8$)Alkyliden-$SO_3^{\ominus}$, $C_3$-$C_5$-Alkenyl, $C_5$-$C_7$-Cycloalkyl, Phenyl, Tolyl, Benzyl oder Glycidyl bedeuten, oder $R_1$ zusammen mit $R_2$ und gegebenenfalls mit $R_3$, zusammen mit dem $\overset{\oplus}{N}$-Atom, an das sie gebunden sind, einen N-Heterocyklus bilden, der gegebenenfalls 1-3 N-Atome oder ein O-Atom als Ringglieder enthält,

R″ Wasserstoff oder $C_1$-$C_4$-Alkyl ist, und

$X^{\ominus}$, falls nicht in $R_1$, $R_2$, $R_3$ oder $R_{33}$ vorhanden, ein farbloses organisches oder anorganisches Anion bedeutet.

Bevorzugt sind Aufzeichnungsmaterialien enthaltend eine Verbindung der Formel I, worin in der Formel I g = 0 ist, und insbesondere worin R33 in der Formel Ia eine Bedeutung von $R_3$ hat. Ganz besonders bevorzugt sind darunter diejenigen, worin die Formel Ia einer Gruppe der Formel If

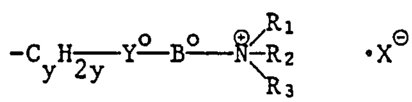
(If)

entspricht, wobei

$Y^o$ -COO-, -CONH- oder

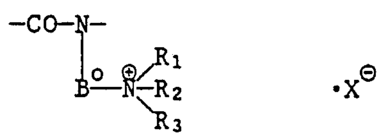

bedeutet, y eine Zahl von 0 bis 6 ist, und p, $B^o$, $R_1$, $R_2$ und $R_3$ die zuvor genannte Bedeutung haben.

Ebenfalls bevorzugt sind Aufzeichnungsmaterialien, worin die Verbindung der Formel I einer Verbindung der Formel II oder III

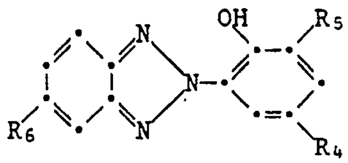
(II)

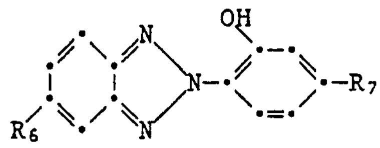
(III)

entspricht, worin
$R_4$ Halogen, $C_1$-$C_4$-Alkoxy, $C_1$-$C_8$-Alkyl, $C_5$-$C_6$-Cycloalkyl, Phenyl, Phenyl-$C_1$-$C_4$-Alkyl oder eine Gruppe der Formel Ia ist,
$R_5$ und $R_6$ unabhängig voneinander Wasserstoff sind, oder eine Bedeutung von $R_4$ haben,
$R_7$ OH, $C_1$-$C_8$-Alkoxy, welches durch 1 bis 3 OH oder 1 bis 2 -COOH substituiert sein kann, $C_1$-$C_8$-Alkanoyloxy, eine Gruppe der Formel Ia mit g = 1 oder Glycidyloxy ist,
unter der Bedingung, dass mindestens eines von $R_4$, $R_5$ und $R_6$ in der Formel II und mindestens eines von $R_6$ und $R_7$ in der Formel III für eine Gruppe der Formel Ia steht.

Besonders bevorzugt aus dieser Gruppe sind diejenigen, worin $R_4$ Halogen, $C_1$-$C_5$-Alkyl, Cyclohexyl oder eine Gruppe der Formel Ia ist, $R_5$ Wasserstoff ist oder eine Bedeutung von $R_4$ hat, $R_6$ für Wasserstoff, Halogen oder eine Gruppe der Formel Ia steht, und $R_7$ OH, gegebenenfalls mit 1 bis 3 OH substituiertes $C_1$-$C_4$-Alkoxy, Glycidyloxy, $C_2$-$C_3$-Alkanoyloxy oder eine Gruppe der Formel Ia mit g = 1 bedeutet.

Ganz besonders bevorzugt sind diejenigen, worin in der Formel II $R_4$ $C_1$-$C_4$-Alkyl oder eine Gruppe der Formel If

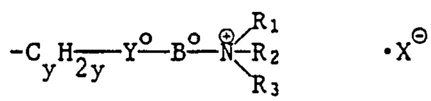
(If)

bedeutet,
$R_5$ Wasserstoff, $C_1$-$C_4$-Alkyl oder eine Gruppe If ist, und $R_6$ Wasserstoff, Cl oder die Gruppe If, worin y für O steht, bedeutet und B°, Y°, $R_1$, $R_2$ und $R_3$ die zuvor genannte Bedeutung haben, unter der Bedingung, dass mindestens eines von $R_4$, $R_5$ und $R_6$ eine Gruppe der Formel If darstellt.

Ebenfalls von Interesse sind Aufzeichnungsmaterialien, worin die Verbindung der Formel I einer Verbindung der Formel V

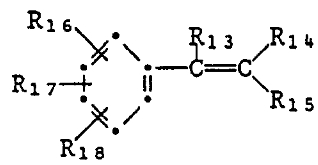
(V)

entspricht, worin
$R_{13}$ Wasserstoff, $C_1$-$C_8$-Alkyl, $C_3$-$C_8$-Alkenyl, Phenyl-$C_1$-$C_4$-Alkyl oder eine Gruppe der Formel

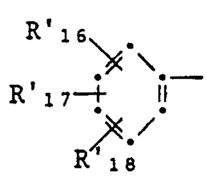

bedeutet, $R_{14}$ und $R_{15}$ unabhängig voneinander -C≡N, -CO-Alkyl($C_1$-$C_4$), -COO-$R_{19}$ oder -CO-$NR_{20}R_{21}$ bedeuten, $R_{16}$, $R_{17}$, $R_{18}$, $R'_{16}$, $R'_{17}$ und $R'_{18}$ unabhängig voneinander für Wasserstoff, $C_1$-$C_4$-Alkyl, Phenyl, Glycidyl, OH, Halogen, gegebenenfalls mit 1 bis 3 OH substituiertes $C_1$-$C_4$-Alkoxy, Phenoxy, Benzyloxy oder eine Gruppe der Formel Ia stehen, und
$R_{19}$, $R_{20}$ und $R_{21}$ unabhängig voneinander Wasserstoff, $C_1$-$C_4$-Alkyl, welches gegebenenfalls mit 1 bis 3 OH substituiert ist, $C_5$-$C_7$-Cycloalkyl, Phenyl, Benzyl, Tolyl, Glycidyl oder eine Gruppe der Formel Ig

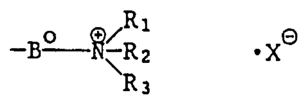 (Ig)

bedeuten, worin B°, $R_1$, $R_2$ und $R_3$ die zuvor genannte Bedeutung haben, unter der Bedingung, dass mindestens eines von $R_{16}$, $R_{17}$, $R_{18}$, $R'_{16}$, $R'_{17}$ oder $R'_{18}$ eine Gruppe der Formel Ia oder mindestens eines von $R_{19}$, $R_{20}$ oder $R_{21}$ eine Gruppe der Formel Ig bedeuten.

Von besonderem Interesse sind Aufzeichnungsmaterialien, worin die Verbindung der Formel V einer Verbindung der Formel Va

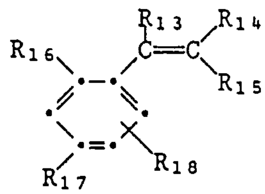 (Va)

entspricht, worin

$R_{13}$ Wasserstoff, $C_1$-$C_4$-Alkyl oder eine Gruppe

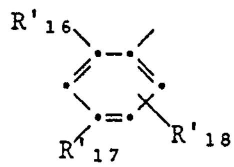

darstellt, und $R_{14}$, $R_{15}$, $R_{16}$, $R_{17}$, $R_{18}$, $R'_{16}$, $R'_{17}$ und $R'_{18}$ die zuvor genannte Bedeutung haben.

Es sind ferner Aufzeichnungsmaterialien bevorzugt, worin die Verbindung der Formel I einer Verbindung der Formel VI

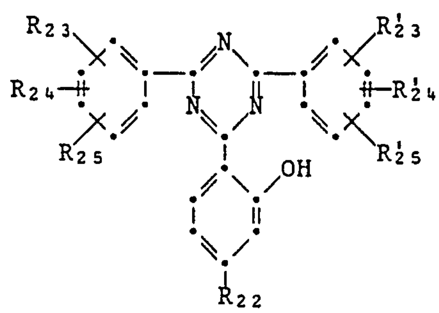 (VI)

entspricht, worin $R_{22}$ eine Gruppe der Formel Ia mit g = 1 ist und $R_{23}$, $R_{24}$, $R_{25}$, $R'_{23}$, $R'_{24}$ und $R'_{25}$ für Wasserstoff, OH, Halogen, $C_1$-$C_4$-Alkoxy, $C_1$-$C_4$-Alkyl, Phenyl oder eine Gruppe der Formel Ia mit g = 1 stehen.

Besonders bevorzugt sind davon Aufzeichnungsmaterialien, worin die Verbindung der Formel VI einer Verbindung der Formel VIa

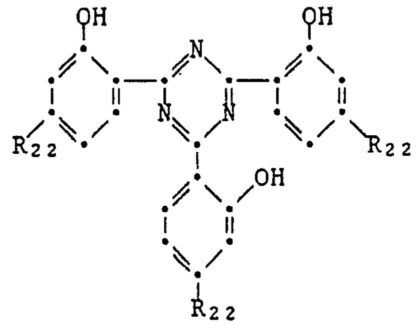

(VIa)

entspricht, und $R_{22}$ für eine Gruppe der Formel Ia mit g = 1 steht.

$X^{\ominus}$ in der Formel Ia bedeutet vorzugsweise Halogen$^{\ominus}$, insbesondere $Cl^{\ominus}$ oder $Br^{\ominus}$, $C_1$-$C_4$-Alkyl-$COO^{\ominus}$, $C_1$-$C_4$-Alkyl-$OSO_3^{\ominus}$ oder $R^o$-$SO_3^{\ominus}$, wobei $R^o$ für Methyl, Tolyl oder -$CF_3$ steht.

Etwaige Alkylreste als $C_1$-$C_4$-Alkyl bedeuten beispielsweise Methyl, Ethyl, n-Propyl, Isopropyl, n-Butyl, sek-Butyl oder t-Butyl.

Etwaige Alkylreste als $C_1$-$C_8$-Alkyl können zusätzlich zu den genannten Bedeutungen beispielsweise n-Pentyl, t-Amyl, n-Hexyl, n-Heptyl, 2-Ethylhexyl, n-Octyl oder 1,1,3,3-Tetramethylbutyl sein.

$R'$ als $C_2$-$C_3$-Hydroxyalkyl bedeutet beispielsweise 2-Hydroxyethyl oder 2-Hydroxypropyl.

Etwaige Reste als gegebenenfalls durch 1 bis 3 OH substituiertes $C_1$-$C_8$-Alkyl können zusätzlich zu den Bedeutungen von $R'$ beispielsweise Hydroxymethyl, 3-Hydroxybutyl, 2,3-Dihydroxypropyl, 2,2-Di-(hydroxymethyl)-propyl, 4-Hydroxybutyl, 6-Hydroxyhexyl, 8-Hydroxyoctyl, 1,2,4-Trihydroxybut-2-yl, 1,2,6-Trihydroxy-hex-2-yl, 1,2,3-Trihydroxy-prop-2-yl sein.

$R_1$, $R_2$ und $R_3$ bzw. $R_{33}$ als durch ein oder mehrere -O- unterbrochenes $C_2$-$C_8$-Hydroxyalkyl bedeuten beispielsweise 4-Hydroxy-3-oxapropyl, 5-Hydroxy-3-oxapentyl, 8-Hydroxy-3,6-dioxaoctyl oder 11-Hydroxy-3,6,9-trioxaundecyl.

B als $C_2$-$C_6$-Alkylen der -Alkyliden bedeutet beispielsweise Ethylen, Ethyliden, Tri-, Tetra-, Penta- oder Hexamethylen, 1,2-Propylen, 2,2-Propyliden, 2,2-Butyliden, 1,2-Butylen oder 2,2-Dimethyl-1,3-propylen.

A als $C_1$-$C_6$-Alkylen oder -Alkyliden kann zusätzlich zu den Bedeutungen von B auch Methylen sein.

Stehen $R_1$, $R_2$ und $R_3$ für -($C_2$-$C_8$)-Alkylen-$SO_3^-$, so kann dem Alkylenrest die Bedeutung von B zukommen und zusätzlich Heptamethylen, Octamethylen oder 3,3-Dimethyl-1,5-pentylen bedeuten.

Etwaige Alkoxyreste können beispielsweise Methoxy, Ethoxy, Isopropoxy, Butoxy, t-Butoxy, n-Hexoxy, 2-Ethylhexyloxy oder n-Octyloxy sein.

Etwaige Phenyl-$C_1$-$C_4$-Alkyl-Reste können beispielsweise Benzyl, 2-Phenylethyl, 1-Phenylethyl, 3-Phenylpropyl oder 2-Phenylpropyl-2 sein.

Bedeutet die Gruppe

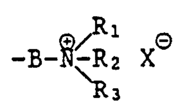

einen N-Heterocyklus, so kommen zum Beispiel folgende Heterocyklen in Frage:

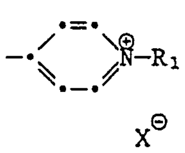, 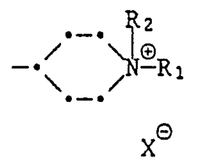, 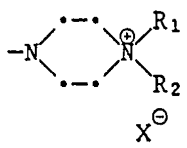, 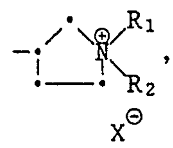, 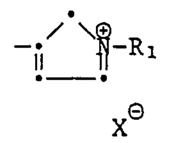,

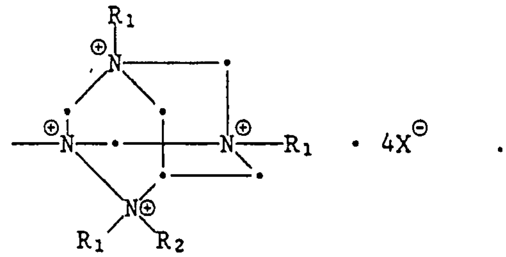 .

Bedeuten $R_1$ und $R_2$, gegebenfalls mit $R_3$ zusammen, einen N-Heterocyklus, so kommen beispielsweise folgende Heterocyklen in Frage:

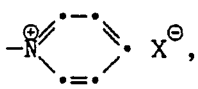, 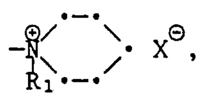, 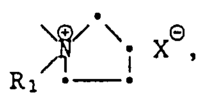, 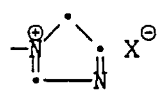

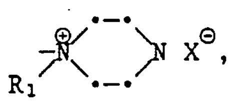, 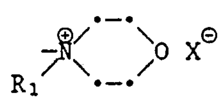 oder 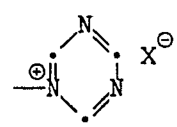 .

Stehen $R_1$, $R_2$ und $R_3$ für ein durch -COOR'' substituiertes $C_1$-$C_8$-Alkyl, so kann für den Alkylteil die Bedeutung von A als $C_1$-$C_6$-Alkylen oder -Alkyliden in Frage kommen und zusätzlich Heptamethylen, Octamethylen, 3-Methyl-1,1-butyliden oder 2-Methyl-1,1-butyliden.

B als ein gegebenenfalls mit OH substituiertes $C_2$-$C_6$-Alkylen oder -Alkyliden, welches gegebenenfalls durch ein -O- oder ein oder zwei - $\overset{\oplus}{N}(R')_2$- unterbrochen ist, bedeutet einen Rest, worin zwischen 2 $X^{\ominus}$-Heteroatomen sich mindestens 2 C-Atome befinden, wie zum Beispiel 3-Oxa-1,5-pentylen, 4-Oxa-1,7-heptylen, 3-Oxa-2,4-dimethyl-1,5-pentylen, 2-Hydroxy-1,3-propylen, 3-Hydroxy-1,5-pentylen, 4-Hydroxy-1,6-hexylen, quaterniertes 3-Aza-1,5-pentylen, 4-Aza-1,7-heptylen, 2,5-Diaza-1,6-hexylen, 2,5-Diaza-1,7-heptylen, 3,5-Diaza-1,6-hexylen oder 3,6-Diaza-1,8-octylen, 4-Oxa-2,6-dihydroxy-1,7-heptylen, 3-Oxa-5-hydroxy-1,6-hexylen, quaterniertes 3,6-Diaza-5-hydroxy-1,8-octylen oder 2-Hydroxy-4-aza-1,7-heptylen.

$R_1$, $R_2$ und $R_3$ als ein durch ein OH substituiertes -($C_1$-$C_8$)-Alkylen-$COO^{\ominus}$ oder -($C_1$-$C_8$)-Alkyliden-$COO^{\ominus}$ bedeuten beispielsweise 1-Hydroxy-3-carboxypropylen, 2-Hydroxy-4-carboxy-butylen, 1-Carboxy-2-hydroxyethyl-1-iden, 1-Carboxy-2-hydroxy-propyl-1-iden oder 2-Hydroxy-8-carboxyoctylen.

$R_1$, $R_2$ und $R_3$ als $C_3$-$C_5$-Alkenyl bedeuten beispielsweise Allyl, Methallyl, n-But-2-enyl, 2-Methyl-prop-2-enyl oder n-Pent-2-enyl. Etwaige $C_3$-$C_8$-Alkenylreste können zusätzlich n-Hexenyl, n-Heptenyl, n-Octenyl oder 2,5-Dimethyl-3-hexenyl sein.

Etwaige Cycloalkylreste als $C_5$-$C_7$-Cycloalkyl bedeuten beispielsweise Cyclopentyl, Cyclooctyl, Cyclohexyl oder Cycloheptyl.

Die folgenden Beispiele für Verbindungen der Formel I illustrieren die Erfindung:

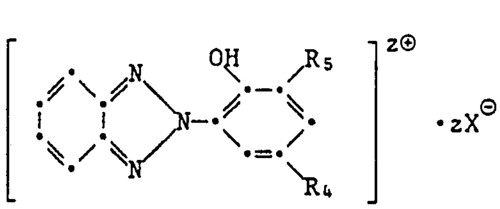

| $R_4$ | $R_5$ | $X^{\ominus}$ |
|---|---|---|
| 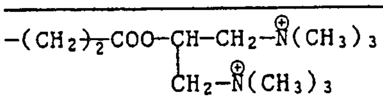 | t-Butyl | $Cl^{-}$ |
| 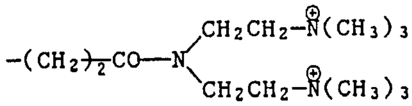 | t-Butyl | $CH_3O-SO_3^{\ominus}$ |
| 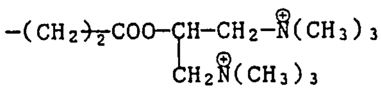 | Methyl | $CH_3CH_2O-SO_3^{\ominus}$ |
| 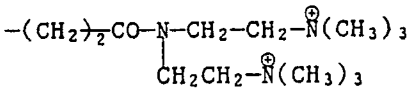 | Methyl | 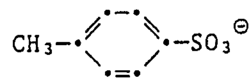 |
| 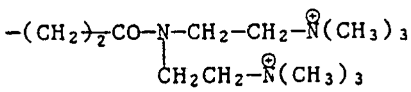 | Methyl | $Cl^{\ominus}$ |
| 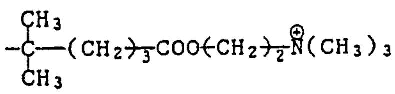 | wie $R_4$ | $CH_3CH_2OSO_3^{\ominus}$ |
| 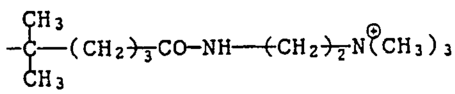 | wie $R_4$ | $CH_3OSO_3^{\ominus}$ |
| 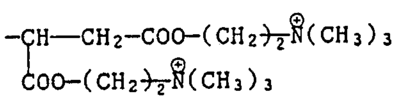 | t-Butyl | $Cl^{\ominus}$ |
| 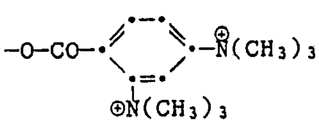 | t-Butyl | $CH_3OSO_3^{\ominus}$ |
| 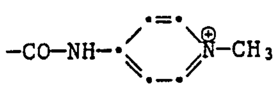 | Methyl | $CH_3CH_2-C^{\ominus}O^{\ominus}$ |
| 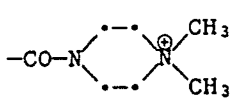 | Methyl | $CH_3-SO_3^{\ominus}$ |
| 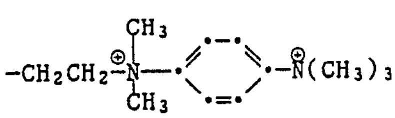 | t-Butyl | $CH_3COO^{\ominus}$ |

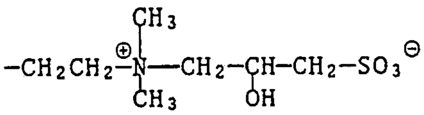 t-Butyl -

Weitere Beispiele:

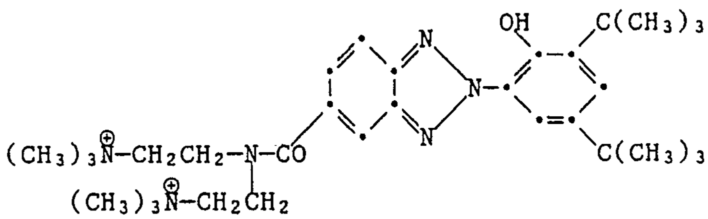

• 2 $CH_3OSO_3^{\ominus}$

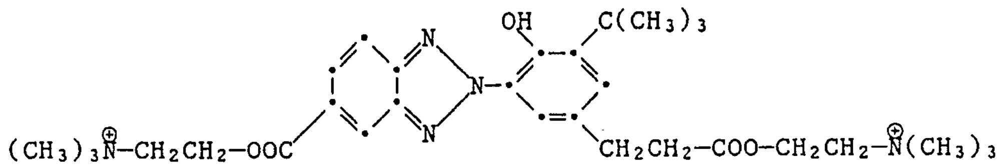

•2 $Cl^-$

Die wasserlöslichen Verbindungen der Formel I können zum Beispiel dadurch hergestellt werden, dass man 1 Moläquivalent einer Verbindung der Formel Ih

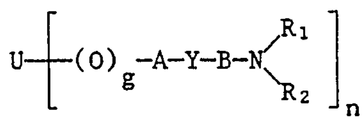 (Ih)

mit n Moläquivalent eines Quaternierungsmittels der Formel $R_{33}$-X quaterniert, wobei U, A, Y, B, $R_1$, $R_2$, $R_{33}$, X, g und n die zuvor genannte Bedeutung haben.

Die Quaternierung kann bei Temperaturen von 0-180 °C, vorzugsweise bei 30-140 °C, durchgeführt werden.

Als Quaternierungsmittel $R_{33}$-X sind beispielsweise folgende Verbindungen geeignet: Alkylhalogenide, wie Methylchlorid, Ethylbromid, Butylbromid oder Benzylchlorid, Dialkylsulfate, wie Dimethyl- oder Diethylsulfat, Sulfonsäureester, wie Toluol- oder Benzosulfonsäuremethyl-oder -ethylester, oder Epichlorhydrin, wobei Alkylchloride besonders bevorzugt werden.

Die Quaternierung mit Alkylhalogeniden, Dialkylsulfaten oder Sulfosäureestern zu den Verbindungen der Formel I wird zweckmässigerweise in einem gegenüber dem Quaternierungsmittel inerten Lösungsmittel oder Lösungsmittelgemisch vorgenommen. Geeignete Lösungsmittel sind z.B. Kohlenwasserstoffe, wie Benzol, Toluol und Xylol, halogenierte aliphatische oder aromatische Kohlenwasserstoffe wie Methylenchlorid, Chloroform, Ethylenchlorid, Chlorbenzol und Dichlorbenzol, Alkohole, wie Ethanol, Butanol und Ethylenglykol, Ether wie Ethylenglykolmonomethylether, Ethylenglykoldimethylether und Dioxan, Amide, wie Dimethylformamid und N-Methylpyrrolidon oder Ketone, wie Aceton.

Die Quaternierung mit Epichlorhydrin wird bei den genannten Temperaturen in saurem Medium, vorteilhaft in Gegenwart einer organischen Säure, wie Ameisensäure, Essigsäure, Propionsäure oder Benzoesäure, durchgeführt, jedoch können auch anorganische Säuren, wie Schwefelsäure, Phosphorsäure oder Halogenwasserstoffsäuren, dafür verwendet werden. Diese anorganischen Säuren können in konzentrierter, handelsüblicher Form als verdünnte wässrige Lösungen oder in Mischung mit den genannten organischen Lösungsmitteln, gegebenenfalls unter Zusatz von Wasser, verwendet werden. Erfolgt die Umsetzung in Gegenwart von organischen Säuren, so wird meistens die konzentrierte Form dieser Säuren angewandt, gegebenenfalls in Mischung mit den genannten organischen Lösungsmitteln.

Die Ausgangsverbindungen der Formel Ih sind teilweise im Handel erhältlich oder können nach bekannten Methoden hergestellt werden.

Die erfindungsgemässen Aufzeichnungsmaterialien eignen sich für druckempfindliche Kopiersysteme, Photokopiersysteme mit Mikrokapseln, wärmeempfindliche Kopiersysteme, photographische Materialien und bevorzugt für den Tintenstrahldruck.

Die erfindungsgemässen Aufzeichnungsmaterialien zeichnen sich durch unerwartete Qualitätsverbesserung aus. Der Tintenstrahldruck kann ohne Gerinnung der Giessmasse erfolgen, bei gleichzeitiger Verbesserung der Lichtechtheit und Waschechtheit.

Die erfindungsgemässen Aufzeichnungsmaterialien besitzen den für die jeweilige Anwendung bekannten Aufbau. Sie bestehen aus einem üblichen Träger, z.B. Papier oder Plastikfolie, der mit einer oder mehreren Schichten beschichtet ist. Diese Schichten enthalten je nach Art des Materials die entsprechend notwendigen Komponenten, wie z.B. im Fall von photographischen Materialien, Silberhalogenidemulsionen, Farbkuppler, Farbstoffe usw. Speziell für Tintenstrahldruck geeignetes Material weist auf einem üblichen Träger eine für Tinte besonders aufnahmefähige Schicht auf.

Unbeschichtetes Papier für Tintenstrahldruck kann ebenfalls eingesetzt werden. Hier dient das Papier gleichzeitig als Trägermaterial und Tintenaufnahmeschicht.

Das Aufzeichnungsmaterial kann auch transparent sein, wie im Falle von Projektionsfolien.

Die Verbindungen der Formel I können bereits bei der Herstellung des Trägermaterials in dieses inkorporiert werden, beispielsweise bei der Herstellung von Papier durch Zusatz in die Papiermasse. Eine zweite Applikationsmethode ist das Besprühen des Trägermaterials mit einer wässrigen Lösung der Verbindungen der Formel I.

Meist wird jedoch eine farbstoffaffine Schicht auf das Trägermaterial aufgebracht und in diesem Fall setzt man die Verbindungen der Formel I dieser Beschichtungsmasse zu. Die Beschichtungsmassen bestehen üblicherweise aus einem festen Füllstoff und einem Bindemittel sowie kleineren Anteilen an Additiven.

Der Füllstoff ist der mengenmässige Hauptbestandteil der Beschichtungsmasse. Beispiele für Füllstoffe, die in Frage kommen, sind Silika, Kaolin, Talk, Ton, Ca-, Mg- oder Al-Silikate, Gips, Zeolith, Bentonit, Diatomenerde, Vermiculit, Stärke oder die in JP-A-60-260 377 beschriebene oberflächenmodifizierte Silika. Kleine Mengen an weissen Pigmenten, wie z.B. Titandioxid, Baryt, Magnesiumoxid, Kalk, Kreide oder Magnesium carbonat können mit dem Füllstoff in der Beschichtungsmasse verwendet werden, sofern sie die Dichte des Tintentstrahldrucks nicht zu stark herabsetzen.

Beschichtungsmassen, die für transparente, projektionsfähige Aufzeichnungsmaterialien bestimmt sind, können keine Licht streuende Teilchen, wie Pigmente und Füllstoffe, enthalten.

Das Bindemittel bindet die Füllstoffe unter sich und an das Trägermaterial. Beispiele für gebräuchliche Bindemittel sind wasserlösliche Polymere, wie z.B. Polyvinylalkohol, partiell hydrolysiertes Polyvinylacetat, Cellulose-ether, Polyvinylpyrrolidon und dessen Copolymere, Polyethylenoxid, Salze von Polyacrylsäure, Natrium-alginat, oxidierte Stärke, Gelatine, Casein, Pflanzengummi, Dextrin, Albumin, Dispersionen von Polyacrylaten oder Acrylat-Methacrylat-Copolymeren, Latices von Natur- oder Synthesekautschuk, Poly(meth)acrylamid, Polyvinylether, Polyvinylester, Copolymere von Maleinsäure, Melaminharze, Harnstoffharze oder chemisch modifizierte Polyvinylalkohole, wie sie in den JP-A-61-134 290 oder 61-134 291 beschrieben sind.

Obwohl die Verbindungen der Formel I dem Tintenstrahldruck Waschechtheit verleihen, kann dem Bindemittel ein zusätzlicher Farbstoffrezeptor oder ein Beizmittel zugesetzt werden, die den Farbstoff fester an die Beschichtung fixieren. Farbstoffrezeptoren für saure Farbstoffe sind kationischer oder amphoterer Natur. Beispiele für kationische Rezeptoren sind polymere Ammoniumverbindungen, wie z.B. Polyvinylbenzyl-trimethyl-ammoniumchlorid, Polydiallyl-dimethylammoniumchlorid, Polymethacryloxyethyl-dimethyl-hydroxyethylammonium-chlorid, Polyvinylbenzyl-methylimidazolium-chlorid, Polyvinylbenzyl-picoliniumchlorid oder Polyvinylbenzyltributylammoniumchlorid. Weitere Beispiele sind basische Polymere, wie z.B. Poly-(dimethylaminoethyl)methacrylat, Polyalkylenpolyamine und deren Kondensationsprodukte mit Dicyandiamid, Amin-Epichlorhydrin-Polykondensate oder die in den JP-A-57-36 692, 57-64 591, 57-187 289, 57-191 084, 58-177 390, 58-208 357, 59-20 696, 59-33 176, 59-96 987, 59-198 188, 60-49 990, 60-71 796, 60-72 785, 60-161 188, 60-187 582, 60-189 481, 60-189 482, 61-14 979, 61-43 593, 61-57 379, 61-57 380, 61-58 788, 61-61 887, 61-63 477, 61-72 581, 61-95 977, 61-134 291 oder in den US-4 547 405 und 4 554 181 sowie in der DE-A-3 417 582 beschriebenen Verbindungen. Ein Beispiel für amphotere Farbstoff-Rezeptoren ist die Gelatine.

Die farbstoffbindende Beschichtung kann eine Reihe weiterer Additive enthalten, wie z.B. Antioxidantien, Lichtschutzmittel (darunter auch UV-Absorber, die nicht den erfindungsgemässen UV-Absorbern angehören), Viskositätsverbesserer, optische Aufheller, Biocide oder/und Antistatika.

Beispiele für geeignete Antioxidantien sind insbesondere sterisch gehinderte Phenole und Hydrochinone, wie z.B. die in der GB-A-2 088 777, oder den JP-A-60-72 785, 60-72 786 und 60-71 796 aufgeführten Antioxidantien.

Beispiele für geeignete Lichtschutzmittel sind insbesondere organische Nickelverbindungen und sterisch gehinderte Amine, wie z.B. die in den JP-A-58-152 072, 61-146 591, 61-163 886, 60-72 785 und 61-146 591 oder die in der GB-A-2 088 777, JP-A-59-169 883 und 61-177 279 erwähnten Lichtschutzmittel.

Der Beschichtungsmasse für Tintenstrahldruck können durchaus weitere UV-Absorber zugefügt werden, so z.B. UV-Absorber, wie sie in der Research Disclosure Nr. 24239 (1984) p. 284, GB-A-2 088 777 und EP-A-0 280 650 beschrieben sind.

In den meisten Fällen kann jedoch, dank der verliehenen hohen Lichtechtheit durch die Verbindungen der Formel I, auf einen zusätzlichen UV-Absorber verzichtet werden.

Um eine gute Dispersion der Füllstoffe und Zusatzstoffe zu gewährleisten, oder um die Giesseigenschaften bzw. die Rheologie der Beschichtungsmasse zu beeinflussen, können letzterer Tenside zugesetzt werden. Als Tenside kommen nur solche nichtionischer, amphoterer oder kationischer Natur in Frage, nicht aber anionische, da die Verbindungen der Formel I selbst kationisch ist.

Beispiele für nicht-ionische Tenside sind Ester oder Ether von Polyethylenoxiden oder Polypropylenoxiden oder von deren Copolymeren, Fettsäurealkanolamide, ethoxylierte Alkanolamide, partielle Fettsäureester von Polyolen (z.B. von Glycerin, Polyglycerin, Sorbit, Pentaerythrit oder Sucrose), N-Alkyl-morpholine oder langkettige Aminoxide.

Beispiele für amphotere Tenside sind Fettsäureamidoalkyl-betaine, Fettsäureamidoalkyl-sultaine, Fettsäure-imidazolin-betaine, N-Alkyl-ß-aminopropionsäuren oder Alkylen-bis(amidoalkylglycinate).

Beispiele von kationischen Tensiden sind die quaternären Ammoniumsalze von langkettigen Fettaminen und Benzylaminen, Imidazolinium-, Pyridinium-, Picolinium- oder Morpholiniumsalze mit langkettigen Alkylresten, quaternäre Ammoniumsalze von langkettigen Alkylamido-alkylaminen oder Bis-ammoniumsalze von quaternären Diaminen.

Die Beschichtungsmasse wird üblicherweise wie folgt vorbereitet: Die wasserlöslichen Komponenten, wie z.B. das Bindemittel, werden in Wasser aufgelöst und zusammengerührt. Die festen Komponenten, wie z.B. Füllstoffe und weitere, bereits beschriebene Zusatzstoffe, werden in diesem wässrigen Medium dispergiert. Das Dispergieren erfolgt vorteilhafterweise mit Hilfe von Geräten, wie z.B. Ultraschall-Proben, Turbo-Rührern, Homogenisatoren, Kolloid-Mühlen, Perlmühlen, Sandmühlen, Hochgeschwindigkeitsrührern und dergleichen.

Es ist ein besonderer Vorteil der Verbindungen der Formel I, dass sie sich leicht in die Beschichtungsmasse einarbeiten lassen. Da sie wasserlöslich sind, können die Verbindungen der Formel I einfach in die Bindemittellösung gegeben werden oder direkt in die Beschichtungsmasse gerührt werden. Der Zerkleinerungsprozess oder Emulgiergang, wie er bei den herkömmlichen UV-Absorbern notwendig ist, entfällt hier.

Die Beschichtungsmasse wird auf den Träger, der meist Papier ist, aufgetragen und durch Erhitzen getrocknet. Die Verbindungen der Formel I können, wie bereits erwähnt, auch in einem separaten Arbeitsgang, allein oder zusammen mit anderen, bereits beschriebenen Komponenten, als wässrige Lösung auf das Aufzeichnungsmaterial gebracht werden. Das Aufbringen kann durch Besprühen, Verleimen in einer Verleimungspresse, einen getrennten Giessvorgang oder durch Eintauchen in eine Wanne erfolgen. Nach einer solchen Nachbehandlung des Aufzeichnungsmaterials ist natürlich ein zusätzlicher Trocknungsvorgang nötig.

Das Verfahren zur Herstellung des gegen Lichtschädigung stabilisierten Aufzeichnungsmaterials für den Tintenstrahldruck ist ebenfalls Gegenstand vorliegender Erfindung, und ist dadurch gekennzeichnet, dass man einen zweidimensionalen Träger mit einer Beschichtungsmasse beschichtet, welche mindestens eine Verbindung der Formel I enthält. Dieses Aufzeichnungsmaterial hat nicht nur ein gutes Aufnahmevermögen für Tintenstrahldruck-Tinten, sie verleiht dem aufgedruckten Farbstoff ausserdem eine hohe Lichtechtheit und Wasserbeständigkeit.

Das so präparierte Aufzeichnungsmaterial enthält vorzugsweise 1 bis 5000 $mg/m^2$, insbesondere 50-1200 $mg/m^2$, einer Verbindung der Formel I.

Bei den erfindungsgemässen Aufzeichnungsmaterialien spielt es keine Rolle, welcher Art die Tinte und der in ihr gelöste Farbstoff ist und welche Art von Druckvorrichtung (printer) verwendet wird.

Bei den heute verwendeten Druckern unterscheidet man solche mit kontinuierlichem Tintenstrahl und drop-on-demand-Drucker, insbesondere bubble-jet-Drucker. Für alle diese apparativen Verfahren lässt sich das erfindungsgemässe Aufzeichnungsmaterial verwenden.

Die Tinten sind meistens wässrige Tinten, sie können aber auch Lösungen des Farbstoffes in einem organischen Lösungsmittel oder in einem geschmolzenen Wachs sein. Wässrige Tinten enthalten meist noch wasserlösliche Lösungsmittel, wie z.B. Mono-, Di-, Tri- oder höhere Ethylenglykole, Propylenglykol, Butandiol-1,4, oder Ether solcher Glykole, Thiodiglykol, Glycerin und dessen Ether und Ester, Polyglycerin, Mono-, Di- und Triethanolamin, Propanolamin, Dimethylformamid, Dimethylsulfoxid, Dimethylacetamid, N-Methylpyrrolidon, 1,3-Dimethylimidazolidon, Methanol, Ethanol, Isopropanol, n-Propanol, Diacetonalkohol, Aceton, Methyl-ethyl-keton oder Propylencarbonat.

Wässrige Tinten enthalten wasserlösliche Farbstoffe, wie sie auch für das Färben von natürlichen

Fasern bekannt sind. Dies können z.B. Monoazo-, Diazo- oder Polyazofarbstoffe oder Reaktivfarbstoffe, Triphenylmethanfarbstoffe, Xanthenfarbstoffe, Phthalocyaninfarbstoffe sein. Beispiele hierfür sind C.I. Food Black 2, C.I. Direct Black 19, C.I. Direct Black 38, C.I. Direct Black 168, C.I. Sulphur Black 1, C.I. Acid Red 35, C.I. Acid Red 249, C.I. Direct Red 227, C.I. Acid Yellow 23, C.I. Direct Yellow 86, C.I. Acid Blue 9, C.I. Direct Blue 86 oder C.I. Direct Blue 199, C.I. Acid Red 14, C.I. Acid Red 52, C.I. Reactive Red 40, C.I. Direct Yellow 107, C.I. Direct Black 154.

Wässrige Tinten können auch verschiedene Additive in kleineren Mengen enthalten, wie z.B. Bindemittel, Tenside, Biocide, Korrosionsinhibitoren, Sequestriermittel, pH-Puffer oder Leitfähigkeitszusätze. Sie können auch anionische wasserlösliche UV-Absorber oder sonstige wasserlösliche Lichtschutzmittel enthalten. Im allgemeinen genügt jedoch die erfindungsgemässe Zugabe eines UV-Absorbers zum Aufzeichnungsmaterial.

Wenn die Tinte eine nicht-wässrige Tinte ist, so stellt sie eine Lösung des Farbstoffes in einem organischen Lösungsmittel oder Lösungsmittelgemisch dar. Beispiele für hierfür verwendete Lösungsmittel sind Alkylcarbitole, Alkylcellosolven, Dialkylformamide, Dialkylacetamide, Alkohole, insbesondere Alkohole mit 1-4 C-Atomen, Aceton, Methyl-ethylketon, Diethylketon, Methyl-isobutylketon, Di-isopropylketon, Dibutylketon, Dioxan, Ethylbutyrat, Ethyl-isovalerat, Diethyl-malonat, Diethylsuccinat, Methyl-pelargonat, Butylacetat, Triethylphosphat, Ethylglykolacetat, Toluol, Xylol, Tetralin, Benzin-Fraktionen. Beispiele für feste Wachse als Lösungsmittel sind Stearin- oder Palmitinsäure.

Solche Tinten auf Lösungsmittel-Basis enthalten darin lösliche Farbstoffe wie z.B. Solvent Rot, Solvent Gelb, Solvent Orange, Solvent Blau, Solvent Grün, Solvent Violett, Solvent Braun oder Solvent Schwarz. Auch solche Tinten können noch weitere Additive enthalten, wie z.B. Bindemittel, Antioxidantien oder Biocide.

Wie bereits erwähnt, umfassen die erfindungsgemässen Aufzeichnungsmaterialien ein weites Feld. Die Verbindungen der Formel I können beispielsweise in druckempfindlichen Kopiersystemen eingesetzt werden. Sie können sowohl in das Papier eingebracht werden, um dort die mikrokapsulierten Farbstoffvorläufer vor Licht zu schützen, als auch in das Bindemittel der Entwicklerschicht, um dort die entstehenden Farbstoffe zu schützen.

Photokopiersysteme mit lichtempfindlichen Mikrokapseln, die mit Druck entwickelt werden, sind in den US-Patentschriften 4 416 966, 4 483 912, 4 532 200, 4 535 050, 4 5365 463, 4 551 407, 4 562 137, 4 608 330 sowie in EP-A-139 479, EP-A-162 664, EP-A- 164-931, EP-A-237 024, EP-A-237 025 oder EP-A-260 129 beschrieben. In all diesen Systemen können die Verbindungen der Formel I in der farbstoffempfangenden Schicht eingesetzt werden. Die Verbindungen der Formel I können aber auch in die Donorschicht eingesetzt werden, um die Farbbildner vor Licht zu schützen.

Photographische Materialien, die stabilisiert werden können, sind photographische Farbstoffe und Schichten, die solche Farbstoffe oder deren Vorprodukte enthalten, beispielsweise photographische Papiere und Filme. Die Verbindungen der Formel I fungieren hier als Antistatika, die die Leitfähigkeit der Gelatineschicht erhöhen, und gleichzeitig als UV-Filter gegen elektrostatische Verblitzungen. In farbphotographischen Materialien werden ausserdem Kuppler und Farbstoffe vor photochemischem Abbau geschützt.

Die Verbindungen der Formel I können ferner in Aufzeichnungsmaterialien, die auf den Prinzipien der Photopolymerisation, Photoplastifizierung oder des Aufbrechens von Mikrokapseln beruhen, oder wo hitze- und licht-empfindliche Diazoniumsalze, Leukofarbstoffe mit Oxidationsmittel oder Farbstofflactone mit Lewis-Säuren Verwendung finden, eingesetzt werden.

Weiterhin können sie in Aufzeichnungsmaterialien für den Farbstoffdiffusionstransfer-, thermischen Wachstransfer- und Punktmatrix-Druck sowie für die Verwendung mit elektrostatischen, elektrographischen, elektrophoretischen, magnetographischen und laserelektrophotographischen Druckern und Feder-Plottern eingesetzt werden.

Die Verbindungen der Formel I können auch in Tinten, vorzugsweise für den Tintenstrahldruck eingesetzt werden. Dabei wird die Wasserbeständigkeit des Tintenstrahldrucks überraschenderweise verbessert. Diese Tatsache ist wahrscheinlich auf die Bildung eines schwerlöslichen Salzes aus dem anionischen Farbstoff und dem kationischen Stabilisator der Formel I zurückzuführen. Es wurde gefunden, dass wässrige Tintenstrahldrucktinten aus einem oder mehreren anionischen Farbstoffen und einer oder mehreren Verbindungen der Formel I hergestellt werden können, insbesondere wenn entweder die anionischen oder die kationischen Komponenten in genügendem Ueberschuss vorhanden sind.

Es ist daher ein weiterer Gegenstand vorliegender Erfindung eine Tinte enthaltend als Stabilisator mindestens eine Verbindung der Formel I.

Die Tinte, insbesondere für den Tintenstrahldruck, enthält bevorzugt Wasser. Ebenfalls bevorzugt sind Tinten, welche den Stabilisator der Formel I in einer Konzentration von 0,01 bis 20 Gew.-%, insbesondere von 0,5 bis 10 Gew.-%, enthalten.

Bevorzugt werden in den erfindungsgemässen Tinten jene Verbindungen der Formel I eingesetzt, die auch für die vorstehend beschriebenen Aufzeichnungsmaterialien als bevorzugt angegeben wurden.

Die Verbindungen der Formel I in den erfindungsgemässen Aufzeichnungsmaterialien sind zum Teil neu und daher ebenfalls Gegenstand vorliegender Erfindung.

Die Erfindung betrifft daher Verbindungen der Formel I°

$U\{SOL^{\circ})_n$ (I°),

worin n eine Zahl von 1 bis 4 bedeutet,

U ein Rest eines UV-Absorbers vom Typ Hydroxyphenyl-benztriazol, Hydroxybenzophenon, Zimtsäure oder Hydroxyphenyl-Triazin ist,

SOL°, gleich oder verschieden, eine Gruppe der Formel I°a

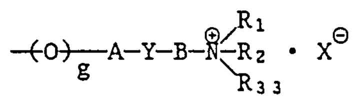 (I°a)

bedeutet, worin

g für 0 oder 1 steht,

A eine direkte Bindung, $C_1$-$C_6$-Alkylen oder eine Gruppe der Formel I°b

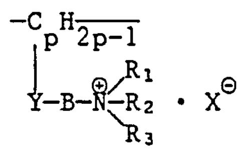 (I°b)

bedeutet, wobei p eine Zahl von 1 bis 6 ist,

Y eine direkte Bindung oder eine der folgenden Gruppen ist:

-CO-, -COO-, -OOC-, -CO-N(R′)-, -(R′)N-CO-, $-SO_2-N(R')-$, $-(R')N-SO_2-$,

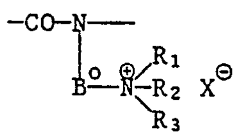 , 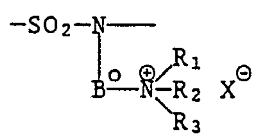 , 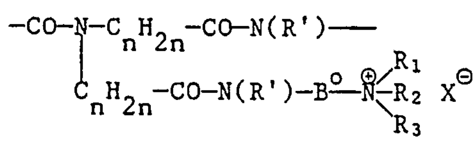 oder

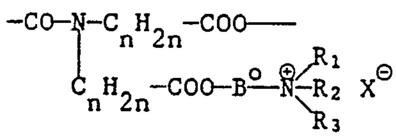 ,

B eine direkte Bindung bedeutet oder ein gegebenenfalls mit OH subituiertes $C_2$-$C_6$-Alkylen ist, welches gegebenenfalls durch ein -O- oder durch ein oder zwei - $\overset{\oplus}{N}X^{\ominus}(R')_2$- unterbrochen ist, oder eine Gruppe der Formeln I°c oder I°C

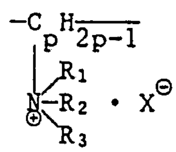 oder 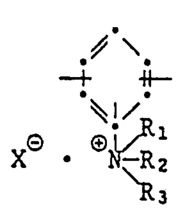

(I°c) (I°C)

ist, oder die Gruppe

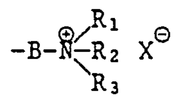

für einen gesättigten oder ungesättigten 1 bis 3-kernigen N-Heterocyklus steht, der 1-4 N-Atome als Ringglieder enthält, von denen mindestens eines quaterniert ist, Heterocyklus bildet, das 1 bis 3 N-Atome enthält, unter den Bedingungen, dass

a) im Falle g = 1, A, Y und B nicht gleichzeitig eine direkte Bindung bedeuten;

b) im Falle g = 1 und A = direkte Bindung, Y -CO- oder eine direkte Bindung bedeutet; und

c) im Falle B = eine direkte Bindung, auch Y eine direkte Bindung ist, R′ Wasserstoff, $C_1$-$C_4$-Alkyl oder $C_2$-$C_3$-Hydroxyalkyl ist,

$B^o$ $-(CH_2)_m-$ oder eine der folgenden Gruppen bedeutet:

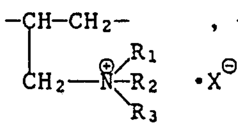 , 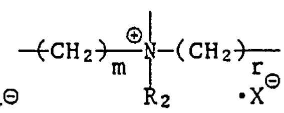 oder 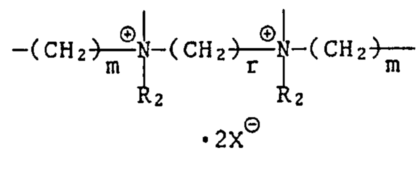 ,

wobei m und r unabhängig voneinander 2 oder 3 bedeuten,

$R_{33}$ eine Bedeutung von $R_3$ hat oder für eine Gruppe der Formeln $I^od$ und $I^oe$

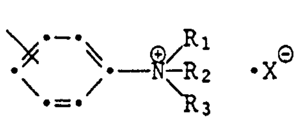

($I^od$)

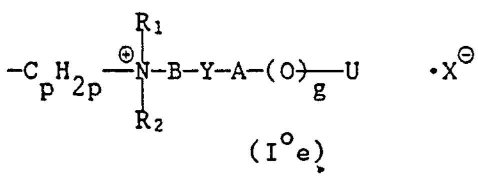

($I^oe$)

steht, worin g, p, B, Y, A und U die zuvor genannte Bedeutung haben, $R_1$, $R_2$ und $R_3$ unabhängig voneinander $C_1$-$C_8$-Alkyl, oder durch ein -COOR″ oder 1 bis 3 OH substituiertes $C_1$-$C_8$-Alkyl, durch ein oder mehrere -O-unterbrochenes $C_2$-$C_8$-Hydroxyalkyl, gegebenenfalls mit einem OH substituiertes -($C_1$-$C_8$)-Alkylen-$COO^{\ominus}$, -($C_1$-$C_8$)Alkyliden-$COO^{\ominus}$, -($C_2$-$C_8$)Alkylen-$SO_3^{\ominus}$ oder -($C_2$-$C_8$)Alkyliden-$SO_3^{\ominus}$ , $C_3$-$C_5$-Alkenyl, $C_5$-$C_7$-Cycloalkyl, Phenyl, Tolyl, Benzyl oder Glycidyl bedeuten, oder $R_1$ zusammen mit $R_2$ und gegebenenfalls mit $R_3$ sowie zusammen mit dem $\overset{\oplus}{N}$-Atom, an das sie gebunden sind, einen N-Heterocyklus bilden, der gegebenenfalls 1-3 N-Atome oder ein O-Atom als Ringglieder enthält,

R″ Wasserstoff oder $C_1$-$C_4$-Alkyl ist, und

$X^{\ominus}$, falls nicht in $R_1$, $R_2$, $R_3$ oder $R_{33}$ vorhanden, ein organisches oder anorganisches Anion bedeutet, unter den Bedingungen, dass

A) im Falle U für einen Rest vom Typ Hydroxyphenyl-Benztriazol steht und n in der Formel $I^o$ 1 bedeutet, Y in der Formel $I^o_a$ die Bedeutung von $Y_1$ hat, wobei $Y_1$ eine der folgenden Gruppen ist:

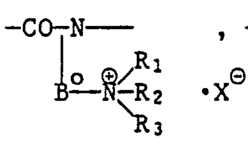 , 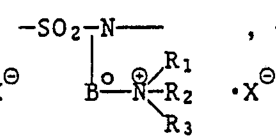 , 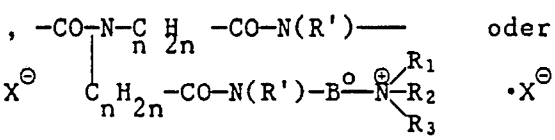 oder

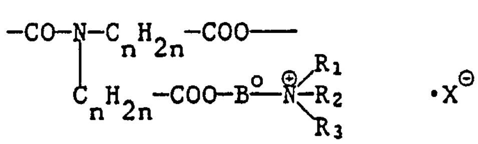 ,

B) im Falle U für einen Rest vom Typ Hydroxybenzophenon steht und n in der Formel $I^o$ 1 oder 2

bedeutet, g = 0 ist;

C) im Falle U für einen Zimtsäurerest mit 1 oder 2 -C≡N Gruppen steht, n in der Formel I° 3 oder 4 ist; oder

D) im Falle U für einen Hydroxyphenyl-Triazinrest steht und in der Formel I°a g = 1, A = Alkylen und Y eine direkte Bindung bedeuten, B nicht für eine direkte Bindung oder Alkylen stehen kann, oder wenn g = 1 und A eine direkte Bindung bedeuten, Y nicht -CO- sein kann.

Die bereits zuvor unter der Formel I gegebenen beispielhaften Bedeutungsmöglichkeiten für $R_1$, $R_2$, $R_3$, R', R'', A, Y und B gelten hier auch.

Bevorzugt sind Verbindungen der Formel I°, die einer Verbindung der Formel II° oder III°

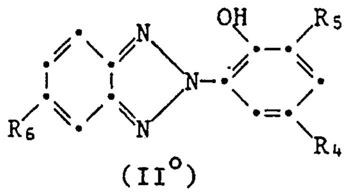

(II°) ,

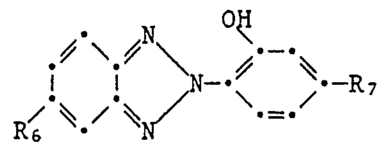

(III°)

entsprechen, worin

$R_4$ Halogen, $C_1$-$C_4$-Alkoxy, $C_1$-$C_8$-Alkyl, $C_5$-$C_6$-Cycloalkyl, Phenyl, Phenyl-$C_1$-$C_4$-Alkyl oder eine Gruppe der Formel I°a ist,

$R_5$ und $R_6$ unabhängig voneinander Wasserstoff sind oder eine Bedeutung von $R_4$ haben,

$R_7$ OH, $C_1$-$C_8$-Alkoxy, welches durch 1 bis 3 OH oder 1 bis 2 -COOH substituiert sein kann, $C_1$-$C_8$-Alkanoyloxy, eine Gruppe der Formel I°a mit g = 1 oder Glycidyloxy ist,

unter der Bedingung, dass mindestens eines von $R_4$, $R_5$ und $R_6$ in der Formel II° und mindestens eines von $R_6$ und $R_7$ in der Formel III für eine Gruppe der Formel I°a steht.

Besonders bevorzugt sind darunter diejenigen Verbindungen der Formel I°, worin $R_4$ Halogen, $C_1$-$C_5$-Alkyl, Cyclohexyl oder eine Gruppe der Formel I°a ist, $R_5$ Wasserstoff ist oder eine Bedeutung von $R_4$ hat, $R_6$ für Wasserstoff, Halogen oder eine Gruppe der Formel I°a steht, und $R_7$ OH, gegebenenfalls mit 1 bis 3 OH oder mit 1 bis 2 COOH substituiertes $C_1$-$C_4$-Alkoxy, Glycidyloxy, $C_2$-$C_3$-Alkanoyloxy oder eine Gruppe der Formel I°a mit g = 1 bedeutet, und insbesondere, worin in der Formel II° $R_4$ $C_1$-$C_4$-Alkyl oder eine Gruppe der Formel I°f

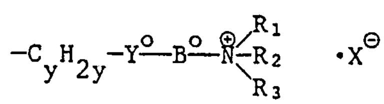

(I°f)

bedeutet,

$R_5$ Wasserstoff, $C_1$-$C_4$-Alkyl oder eine Gruppe I°f ist, und $R_6$ Wasserstoff, Cl oder eine Gruppe I°f bedeutet, worin y für 0 steht, unter der Bedingung, dass mindestens eines von $R_4$, $R_5$ und $R_6$ eine Gruppe I°f darstellt.

Von Interesse sind auch VBerbindungen der Formel I°, die einer Verbindung der Formel V°

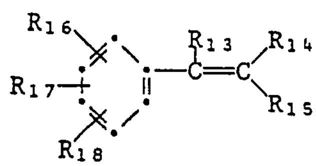

(V°)

entsprechen, worin

$R_{13}$ Wasserstoff, $C_1$-$C_8$-Alkyl, $C_3$-$C_8$-Alkenyl, Phenyl-$C_1$-$C_4$-Alkyl oder eine Gruppe der Formel

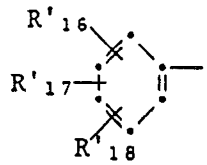

bedeutet, $R_{14}$ und $R_{15}$ unabhängig voneinander -C≡N, -CO-$C_1$-$C_4$-Alkyl, -COO-$R_{19}$ oder -CO-$NR_{20}R_{21}$ bedeuten, $R_{16}$, $R_{17}$, $R_{18}$, $R'_{16}$, $R'_{17}$ und $R'_{18}$ unabhängig voneinander für Wasserstoff, $C_1$-$C_4$-Alkyl, Phenyl, Glycidyl, OH, Halogen, gegebenenfalls mit 1 bis 3 OH substituiertes $C_1$-$C_4$-Alkoxy, Phenoxy, Benzyloxy oder eine Gruppe der Formel I°a stehen, und

$R_{19}$, $R_{20}$ und $R_{21}$ unabhängig voneinander Wasserstoff, $C_1$-$C_4$-Alkyl, welches gegebenenfalls mit 1 bis 3 OH substituiert ist, $C_5$-$C_7$-Cycloalkyl, Phenyl, Benzyl, Tolyl, Glycidyl oder eine Gruppe der Formel I°g

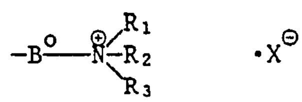

(I°g)

bedeuten, worin B°, $R_1$, $R_2$ und $R_3$ die zuvor genannte Bedeutung haben, unter der Bedingung, dass mindestens eines von $R_{16}$, $R_{17}$, $R_{18}$, $R'_{16}$, $R'_{17}$ oder $R'_{18}$ eine Gruppe der Formel I°a und mindestens eines von $R_{19}$, $R_{20}$ oder $R_{21}$ eine Gruppe der Formel I°g bedeuten, und falls $R_{14}$ und/oder $R_{15}$ für -C≡N stehen, mindestens 3 Gruppen der Formel I°a und/oder I°g sind vorhanden sind.

Von besonderem Interesse sind davon diejenigen, die einer Verbindung der Formel V°a

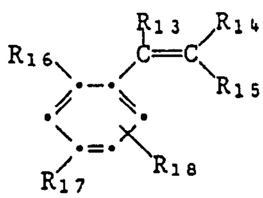

(V°a)

entsprechen, worin

$R_{13}$ Wasserstoff, $C_1$-$C_4$-Alkyl oder eine Gruppe

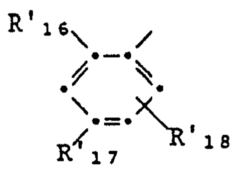

darstellt, und $R_{14}$, $R_{15}$, $R_{16}$, $R_{17}$, $R_{18}$, $R'_{16}$, $R'_{17}$ und $R'_{18}$ die zuvor genannte Bedeutung haben.

Ebenfalls von Interesse sind Verbindungen der Formel I°, die einer Verbindung der Formel VI°

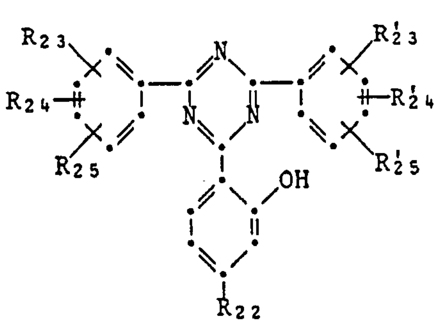

(VI°)

entsprechen, worin

$R_{22}$ eine Gruppe der Formel I°a mit g = 1 ist und $R_{23}$, $R_{24}$, $R_{25}$, $R_{23}'$, $R_{24}'$ und $R_{25}'$ für Wasserstoff, OH, Halogen, $C_1$-$C_4$-Alkoxy, $C_1$-$C_4$-Alkyl, Phenyl oder eine Gruppe der Formel I°a mit g = 1 stehen, unter der Bedingung, dass falls in der Formel I°a g = 1, A = Alkylen und Y eine direkte Bindung bedeuten, B nicht für eine direkte Bindung oder Alkylen steht, oder falls g = 1 und A eine direkte Bindung bedeuten, Y nicht -CO- sein kann.

Von besonderem Interesse sind davon diejenigen, die einer Verbindung der Formel VI°a

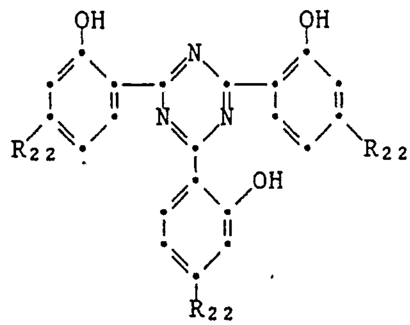

(VI°a)

entsprechen, und $R_{22}$ für eine Gruppe der Formel I°a mit g = 1 steht.

Es werden ferner Verbindungen der Formel I° bevorzugt, worin $X^{\ominus}$ in der Formel I°a $Halogen^{\ominus}$, $C_1$-$C_4$-$Alkyl-COO^{\ominus}$, $C_1$-$C_4$-$Alkyl-OSO_3^{\ominus}$ oder $R^o$-$SO_3^{\ominus}$ bedeutet, wobei $R^o$ für Methyl, Tolyl oder -$CF_3$ steht.

Die neuen Verbindungen der Formel I° können, wie bereits beschrieben, nach an sich bekannten Methoden hergestellt werden.

Die neuen Verbindungen der Formel I° können als Stabilisatoren für organische Materialien, insbesondere gegen deren Schädigung durch Licht, Sauerstoff und Wärme, verwendet werden. Die zu stabilisierenden Materialien können z.B. Oele, Fette, Wachse, Kosmetika, Farbstoffe oder Polymere sein. Auf Grund ihrer Struktur sind sie vorteilhaft für die kathodische elektrophoretische Beschichtung sowie in Lacken und Schutzanstrichen auf Wasserbasis.

Die neuen Verbindungen der Formel I° werden bevorzugt als Stabilisatoren für Aufzeichnungsmaterialien oder Tinten für den Tintenstrahldruck verwendet.

Beispiele von Polymeren, die mit den erfindungsgemässen Verbindungen der Formel I vorteilhaft stabilisiert werden können, sind:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-(α-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyarylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol. .

6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von α,ß-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso-und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von

Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die erfindungsgemässen Stabilisatoren werden den Polymeren zweckmässig in einer Konzentration von 0,01-10 Gew.-%, berechnet auf das zu stabilisierende Material, zugesetzt. Vorzugsweise werden 0,05 bis 5,0 Gew.-%, besonders bevorzugt 0,1 bis 2,0 Gew.-% der Verbindungen der Formel I, berechnet auf das zu stabilisierende Material, in dieses eingearbeitet.

Die Einarbeitung kann beispielsweise durch Einmischen der erfindungsgemässen Stabilisatoren und gegebenenfalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels, erfolgen. Die erfindungsgemässen Stabilisatoren können auch in Form eines Masterbatches, der den Stabilisator beispielsweise in einer Konzentration von 2,5 bis 25 Gew.-% enthält, den zu stabilisierenden Kunststoffen zugesetzt werden.

Die Verbindungen der Formel I° können auch vor oder während der Polymerisation oder der Vernetzungsreaktion zugefügt werden. Man erhält so direkt stabilisierte Polymere.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Besonders bevorzugt ist die Verwendung der erfindungsgemässen Verbindungen in Lacken aller Art. Dies können pigmentierte oder unpigmentierte Lacke oder Metalleffektlacke sein. Sie können ein organisches Lösungsmittel enthalten oder lösungsmittelfrei sein oder wässrige Lacke sein. Von besonderer Bedeutung ist die Verwendung von wässrigen Lacken, wie sie beispielsweise für elektrophoretische Beschichtungen oder für Holzlackierungen verwendet werden.

Die Lacke können als Bindemittel mindestens eines der vorhin aufgeführten Polymeren enthalten. Beispiele für Lacke mit speziellen Bindemitteln sind die folgenden:

1. Lacke auf Basis von kalt- oder heiss-vernetzbaren Alkyd-, Acrylat-, Polyester-, Epoxid- oder Melaminharzen oder Mischungen solcher Harze, gegebenenfalls mit Zusatz eines sauren Härtungskatalysators;

2. Zweikomponenten-Polyurethanlacke auf Basis von hydroxylgruppenhaltigen Acrylat-, Polyester- oder Polyetherharzen und aliphatischen oder aromatischen Polyisocyanaten;

3. Einkomponenten-Polyurethanlacke auf Basis von blockierten Polyisocyanaten, die während des Einbrennens deblockiert werden;

4. Zweikomponentenlacke auf Basis von (Poly)ketiminen und aliphatischen oder aromatischen Polyisocyanaten;

5. Zweikomponentenlacke auf Basis von (Poly)ketiminen und einem ungesättigten Acrylatharz oder einem Polyacetoacetatharz oder einem Methylacrylamidoglykolat-methylester;

6. Zweikomponentenlacke auf Basis von carboxyl- oder aminogruppenhaltigen Polyacrylaten und Polyepoxiden;

7. Zweikomponentenlacke auf Basis von anhydridgruppenhaltigen Acrylatharzen und einer Polyhydroxy- oder Polyaminokomponente;

8. Zweikomponentenlacke auf Basis von (Poly)oxazolidinen und anhydridgruppenhaltign Acrylatharzen oder ungesättigten Acrylatharzen oder aliphatischen oder aromatischen Polyisocyanaten.

9. Zweikomponentenlacke auf Basis von ungesättigten Polyacrylaten und Polymalonaten;

10. thermoplastische Polyacrylatlacke auf Basis von thermoplastischen Acrylatharzen oder fremdvernetzenden Acrylatharzen in Kombination mit veretherten Melaminharzen;

11. Lacksysteme auf Basis von siloxanmodifizierten Acrylatharzen.

Die Lacke können auch strahlenhärtbare Lacke sein. In diesem Fall besteht das Bindungsmittel aus monomeren oder oligomeren Verbindungen, die ethylenische Doppelbindungen enthalten und durch Bestrahlung mit aktinischem Licht oder mit Elektronenstrahlen in eine vernetzte hochmolekulare Form übergehen. Meist handelt es sich hierbei um ein Gemisch solcher Verbindungen.

Die Lacke können als Einschicht- oder Zweischichtlacke appliziert werden, wobei die erfindungsgemässen Stabilisatoren vorzugsweise der unpigmentierten obersten Schicht zugesetzt werden. Zusätzlich zu den erfindungsgemässen Stabilisatoren können solche Lacke auch die in den folgenden Abschnitten 1. bis 10. aufgezählten Additive enthalten. Insbesondere zeigen Kombinationen dieser Stabilisatoren mit den unter 2.7. genannten sterisch gehinderten Aminen gute Stabilisierungseffekte.

Die Lacke können auf die Substrate (Metall, Plastik, Holz etc.) nach den üblichen Verfahren aufgebracht werden, beispielsweise durch Streichen, Besprühen, Giessen, Tauchen oder Elektrophorese.

In der Praxis können die erfindungsgemässen Stabilisatoren zusammen mit anderen Stabilisatoren eingesetzt werden.

Als weitere Additive, mit denen zusammen die erfindungsgemäss verwendeten Stabilisatoren eingesetzt werden können, sind beispielsweise zu nennen:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Dicyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Di-phenyl-4-octadecyloxyphenol.

1.3. Hydroxylierte Thiodiphenylether, z.B. 2,2′-Thio-bis-(6-tert.-butyl-4-methylphenol), 2,2′-Thio-bis-(4-octylphenol), 4,4′-Thio-bis-(6-tert.butyl-3-methylphenol), 4,4′-Thio-bis-(6-tert.butyl-2-methylphenol).

1.4. Alkyliden-Bisphenole, z.B. 2,2′-Methylen-bis-(6-tert.butyl-4-methylphenol), 2,2′-Methylen-bis-(6-tert.butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert.butylphenol), 2,2′-Ethyliden-bis-(4,6-di-tert.butylphenol), 2,2′-Ethyliden-bis-(6-tert.butyl-4-isobutylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert.-butylphenol), 4,4′-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3′-tert.butyl-4′-hydroxyphenyl)-butyrat], Bis-(3-tert.butyl-4-hydroxy-5-methylphenyl)-dicyclopentadien, Bis-[2-(3′-tert.butyl-2′-hydroxy-5′-methylbenzyl)-6-tert.butyl-4-methyl-phenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylesters, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der ß-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N′-Bis-(hydroxyethyl)-oxalsäurediamid.

1.8. Ester der ß-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.9. Ester der β-(3,5-Dicyclohexyl-4-hydroxyphenyl)-propionsäure mit ein-oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.10. Amide der β-(3,5-Di-tert.butyl-4-hydroxyphenyl)-propionsäure, wie z.B. N,N′-Bis-(3,5-di-tert.butyl-4-hydroxyphenylpropionyl)-hexamethylendiamin, N,N′-Bis-(3,5-di-tert.butyl-4-hydroxyphenylpropionyl)-trimethylendiamin, N,N′-Bis-(3,5-di-tert.butyl-4-hydroxyphenylpropionyl)-hydrazin.

2. Weitere UV-Absorber und Lichtschutzmittel

2.1. 2-(2′-Hydroxyphenyl)-benztriazole, wie z.B. das 5′-Methyl-, 3′,5′-Di-tert.butyl-, 5′-tert.Butyl-, 5′-(1,1,3,3-Tetramethylbutyl)-, 5-Chlor-3′,5′-di-tert.butyl-, 5-Chlor-3′-tert.butyl-5′-methyl-, 3′-sec.Butyl-5′-tert.butyl, 4′-Octoxy-, 3′,5′-Di-tert.amyl-, 3′,5′-Bis-(α,α-dimethylbenzyl)-Derivat. Ferner Mischungen aus β-[3-(2-H-benztriazol-2-yl)-4-hydroxy-5-t-butylphenyl]propionsäure-Polyethylenglykol 300-Ester und Bis-{β-[3-(2-H-benztriazol-2-yl)-4-hydroxy-5-t-butylphenyl]propionsäure}-Polyethylenglykol 300-Ester.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2′,4′-Trihydroxy-, 2′-Hydroxy-4,4′-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-ß,ß-diphenylacrylsäure-ethylester bzw. -isooctylester, α-Carbomethoxy-zimtsäuremethylester, α-Cyano-ß-methyl-p-methoxy-zimtsäuremethylester bzw. -butylester, α-Carbomethoxy-p-methoxy-zimtsäure-methylester, N-(ß-Carbomethoxy-ß-cyanovinyl)-2-methyl-indolin.

2.5. Nickelverbindungen, wie z.B. Nickelkomplexe des 2,2′-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis-(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.

2.7. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N′-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1′-(1,2-Ethandiyl)-bis-(3,3,5,5-tetramethyl-piperazinon).

2.8. Oxalsäurediamide, wie z.B. 4,4′-Di-octyloxy-oxanilid, 2,2′-Di-octyloxy-5,5′-di-tert.butyl-oxanilid, 2,2′-Di-dodecyloxy-5,5′di-tert.butyloxanilid, 2-Ethoxy-2′-ethyl-oxanilid, N,N′-Bis-(3-dimethylaminopropyl)-oxalamid, 2-Ethoxy-5-tert.butyl-2′-ethyloxanilid und dessen Gemisch mit 2-Ethoxy-2′-ethyl-5,4′-di-tert.butyl-oxanilid, Gemische von o- und p-Methoxy- sowie von o- und p-Ethoxy-di-substituierten Oxaniliden.

2.9.1. Kationische UV-Absorber, die mindestens eine quaternäre Ammoniumgruppe enthalten, z.B. solche, die in den JP-A-61-19278, 61-192 778, 61-192 780 und FR-A-1 464 919 beschrieben sind.

2.9.2. Anionische UV-Absorber und deren Salze, wie sie z.B. in den JP-A-54-085 804, 54-068 303, 62-106 971, 63-046 277, 59-027 972 und 59-169 883 beschrieben sind.

3. Metalldesaktivatoren, wie z.B. N,N′-Diphenyloxalsäurediamid, N-Salicylal-N′-salicyloylhydrazin, N,N′-Bis-(salicyloyl)-hydrazin, N,N′-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4′-biphenylen-diphosphonit, 3,9-Bis-(2,4-di-tert.butylphenoxy)-2,4,8,10-tetraoxa-3,9-diphosphaspiro[5.5]-undecan.

5. Peroxidzerstörende Verbindungen, wie z.B. Ester der ß-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrittetrakis-(ß-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die nachfolgenden Beispiele erläutern die Erfindung weiter. Etwaige Prozentangaben beziehen sich auf das Gewicht, sofern nichts anderes angegeben ist.

## Herstellungsbeispiele

### Beispiel 1:

a) Herstellung von [1,3-Bis(N,N-dimethylamino)-2-propyl]-3-[3-(2-benztriazolyl)-4-hydroxy-5-t-butylphenyl]-propionat

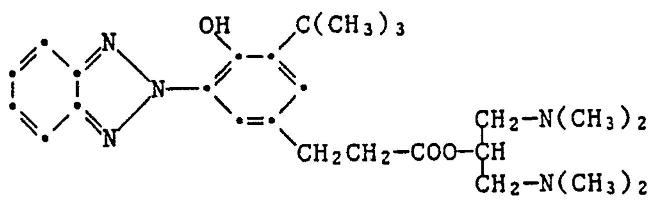

100 g 3-[3-(2-benztriazolyl)-4-hydroxy-5-t-butylphenyl]-propionsäure werden in 600 ml Toluol suspendiert. Man fügt 10 ml Dimethylformamid (DMF) dazu und erwärmt das Gemisch auf 70° C. Anschliessend werden 70,1 g Thionylchlorid tropfenweise dem Gemisch zugefügt. Man beobachtet dabei HCl- und $SO_2$-Entwicklung. Die Lösung wird während 4 Stunden bei 80° C gerührt. Zum Schluss wird der überschüssige Thionylchlorid durch Destillation entfernt. 50,9 g 1,3-Bis(N,N-dimethylamino)-propan-2-ol werden der Lösung bei 80° C tropfenweise zugefügt, wobei HCl-Entwicklung entsteht.

Das Reaktionsgemisch wird 8 Stunden lang bei 80° C gerührt und anschliessend auf 10° C abgekühlt. Der HCl-Niederschlag wird filtriert, mit Ether ausgewaschen, dann in 1 ℓ Wasser gelöst und mit 10 ml conc. $NH_3$ ($NH_3$ mit 25 % $H_2O$) neutralisiert. Das Produkt wird mit Methylenchlorid extrahiert.

Die organische Phase, welche das Rohprodukt enthält, wird mit wässriger NaCl-Lösung gewaschen, über $MgSO_4$ getrocknet und eingeengt. Der Rückstand wird aus einem Petroleumether/Ethylacetat-Gemisch umkristallisiert.

Man erhält 54,4 g von [1,3-Bis(N,N-dimethylamino)-2-propyl]-3-[3-(2-benztriazolyl)-4-hydroxy-5-t-butylphenyl]-propionat als weisse Kristalle (Smp. 79-80° C).

b) Herstellung von [1,3-Bis(N,N,N-trimethylammonium)-2-propyl]-3-[3-(2-benztriazolyl)-4-hydroxy-5-t-butylphenyl]-propionat-di-monomethyl-sulfat

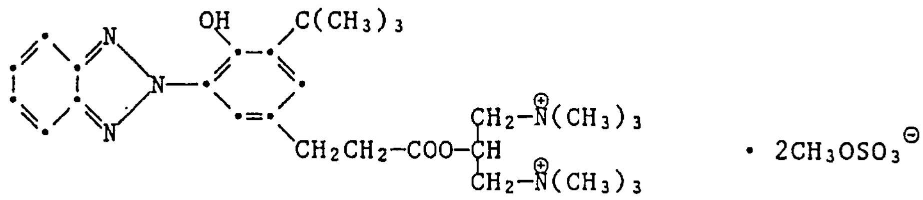

20 g [1,3-Bis(N,N-dimethylamino)-2-propyl]-3-[3-(2-benztriazolyl)-4-hydroxy-5-t-butylphenyl]-propionat werden in 200 ml Aceton gelöst und mit 21,6 g Dimethylsulfat tropfenweise versetzt. Eine exotherme Reaktion findet statt, wobei ein weisser Niederschlag entsteht. Das Reaktionsgemisch wird während einer Stunde bis zum Rückfluss erhitzt. Anschliessend wird der Niederschlag filtriert und mit Aceton gewaschen. Nach der Umkristallisation aus Methanol erhält man 17,4 g von [1,3-Bis(N,N,N-trimethylammonium)-2-propyl]-3-[3-(2-benztriazol)-4-hydroxy-5-t-butylphenyl]-propionat-di-monomethylsulfat als weisse Kristalle (Smp. 196-203 °C).

Beispiel 2: [1,3-Bis(N,N,N-trimethylammonium)-2-propyl]-3-[3-(2-benz-triazol)-4-hydroxy-5-t-butylphenyl]-propionat-dichlorid

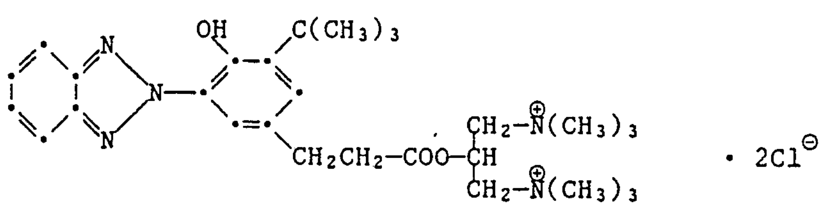

20 g [1,3-Bis(N,N-dimethylamino)-2-propyl]-3-[3-(2-benztriazolyl)-4-hydroxy-5-t-butylphenyl]-propionat werden in 360 ml Aceton/Methylenchlorid (4:1 Volumenteile) gelöst. Es wird Methylchlorid während 30 Minuten durch die Lösung geleitet, anschliessend wird die Flasche verschlossen und über Nacht stehengelassen. Der entstehende Niederschlag wird filtriert, mit ca. 100 ml Petroleumether gewaschen und getrocknet. Nach der Umkristallisation aus einem Methylenchlorid/Petroleumether-Gemisch erhält man 24,0 g von [1,3-Bis(N,N,N-trimethylammonium)-2-propyl]-3-[3-(2-benztriazolyl)-4-hydroxy-5-t-butylphenyl]-propionat-dichlorid als weisse Kristalle (Smp. 105-110 °C, mit Zersetzung verbunden).

Beispiel 3:

a) Herstellung von 3,5-Bis{5′-[2″-(N,N-dimethylamino)-ethoxycarbonyl]-2′-methylpent-2′-yl}-2-hydroxyphenyl-benztriazol

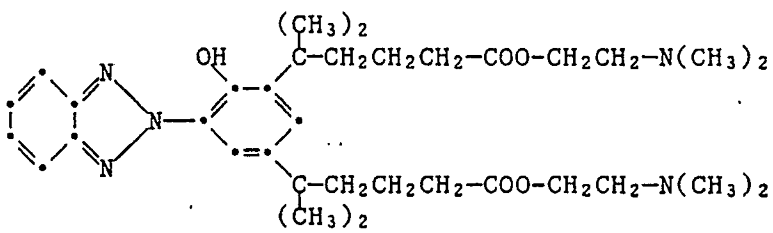

Man geht wie im Beispiel 1a) vor, jedoch verwendet man 23,4 g (0,05 Mol) der entsprechenden Dicarbonsäure. Man erhält 33,5 g (0,049 Mol) des Bishydrochlorids (98 % der Theorie; Smp. 195-198 °C). Anschliessend wird das Produkt neutralisiert und chromatographisch gereinigt (Alex-Typ E mit Acetessigester/Petroleumether in 1:3 Volumenverhältnis). Man erhält 23,5 g (0,039 Mol) des Diamins als

leicht gelbliches Oel (80 % der Theorie).

| Analyse | % C | % H | % N |
|---|---|---|---|
| berechnet: | 66,97 | 8,43 | 11,48 |
| gefunden: | 66,90 | 8,51 | 11,39 |

b) Herstellung von 3,5-Bis{5′-[2″-(N,N,N-trimethylammonium)-ethoxycarbonyl]-2′-methylpent-2′-yl}-2-hydroxyphenyl-benztriazol-bis-monomethylsulfat

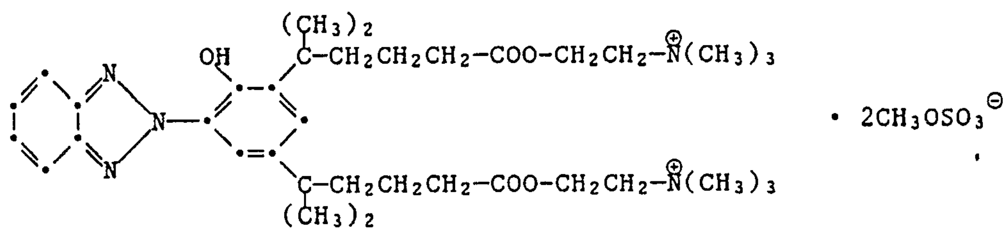

Man verfährt analog Beispiel 1b), wobei 3,0 g des Produktes aus Beispiel 1a) eingesetzt wird. Man erhält 3,5 g der gewünschten Substanz als weisse Kristalle (Smp. 50-65 °C)

Beispiel 4: Herstellung von [1,3-Bis(N,N,N-trimethylammonium)-2-propyl]-3-[3-(5-chloro-2-benztriazolyl)-4-hydroxy-5-t-butylphenyl]propionat-dijodid

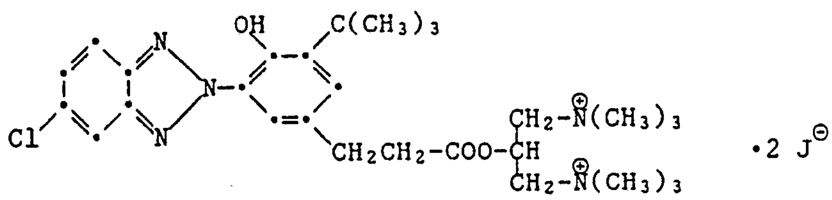

Man verfährt analog den Beispielen 1 und 2, wobei jedoch 100 g der entsprechenden Carbonsäure und anstelle von Methylchlorid Methyljodid verwendet werden. Man erhält 43 g des Produktes als leicht gelbliche Kristalle (Smp. 180-187 °C).

Beispiel 5: Herstellung von {3-[4-(2-benztriazolyl)-3-hydroxyphenoxy]-2-hydroxy-1-propyl}-N,N,N-trimethylammonium-chlorid

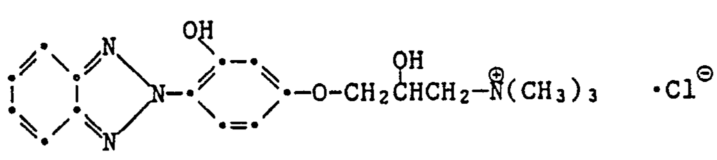

11,4 g (0,05 Mol) 2-(2,4-Dihydroxyphenyl)benztriazol werden mit 7,75 g (0,051 Mol) Glycidyltrimethylammonium-chlorid in 100 ml Acetonitril 24 Stunden lang bei 50-60 °C erwärmt.

Der Niederschlag wird filtriert und in 20 ml Acetonitril zum Rückfluss erhitzt. Nach dem Abkühlen und Filtrieren erhält man 3,7 g eines hellgelben Feststoffes (Smp. 225-228 °C).

Beispiel 6: Herstellung von [1,3-Bis(N,N,N-trimethylammonium)-2-propyl]-2-cyano-3,3-diphenylacrylat-bis-

monomethylsulfat

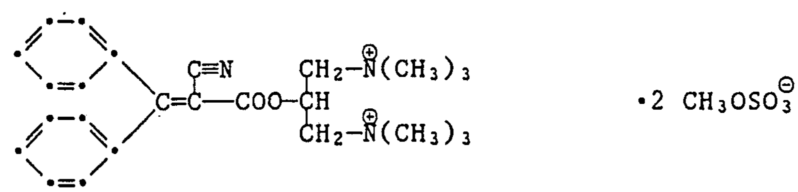

Man verfährt analog Beispiel 1, wobei jedoch 12,5 g 2-Cyano-3,3-diphenylacrylsäure verwendet werden. Man erhält 7,1 g des Produktes als weisse Kristalle (Smp. 189-191 °C).

Beispiel 7: Herstellung von 2-[3,5-Ditertbutyl-2-hydroxyphenyl]-5-benztriazolcarbonsäure-2-N,N-dimethylaminoäthylester

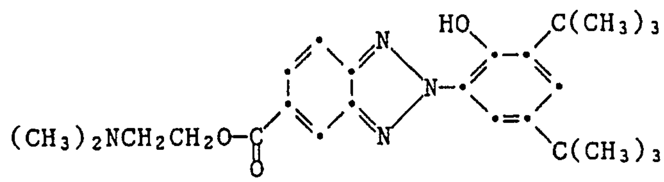

Man verfährt analog Beispiel 1, wobei jedoch 36,7 g 2-[3,5-Ditertbutyl-2-hydroxyphenyl]-5-benztriazolcarbonsäure und 9,8 g 2-N,N-Dimethylaminoäthanol verwendet werden. Man erhält 37,0 g (89 % der Theorie) des Produkts in Form leicht gelblicher Kristalle (Smp. 127 - 129 °C).

Beispiel 8: Herstellung von 2-[3,5-Ditertbutyl-2-hydroxyphenyl]-5-[2-(N-2-hydroxypropyl-N,N-dimethylammonium)-ethoxycarbonyl]benztriazolbromid

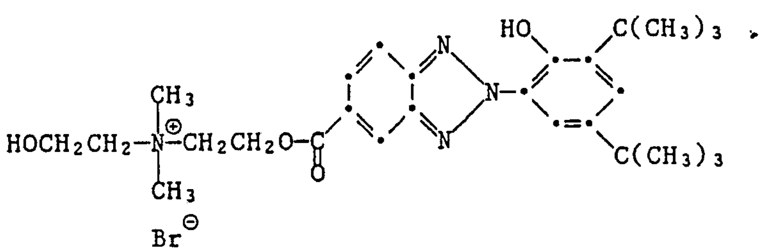

17,5 g 2-[3,5-Ditertbutyl-2-hydroxyphenyl]-5-benztriazolcarbonsäure-2-N,N-dimethylaminoäthylester werden in 70 ml Toluol gelöst. 25 g 2-Bromäthanol werden zugegeben und die Lösung 15 Stunden unter Rückfluss gekocht. Danach lässt man abkühlen, wobei das Produkt ausfällt. Man filtriert, wäscht mit Diäthyläther und trocknet im Vakuum. Man erhält 17,0 g (75 % der Theorie) des Produktes in Form gelblicher Kristalle (Smp. 222 - 224 °C).

Beispiel 9: Herstellung von [N,N-Bis-2-chloräthyl]-3-[3-(2-benztriazolyl)-4-hydroxy-5-t-butylphenyl)-propionamid

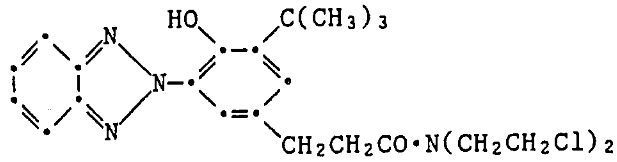

Man verfährt analog Beispiel 1, wobei jedoch 33,9 g 3-[3-(2-Benztriazolyl)-4-hydroxy-5-t-butylphenyl]-propionsäure und 19,6 g Bis(2-Chloräthyl)amin verwendet werden. Das Produkt wird chromatographisch (Alex-Typ E, Essigester/Petroläther 1:4 v/v) gereinigt. Man erhält 5 g des Produktes in Form weisser Kristalle (Smp. 90 - 93 °C).

Beispiel 10: Herstellung von [N,N-Bis[(N,N,N-Trimethylammonium)-2-äthyl]-3-[3-(2-benztriazolyl)-4-hydroxy-5-t-butylphenyl]propionandichlorid

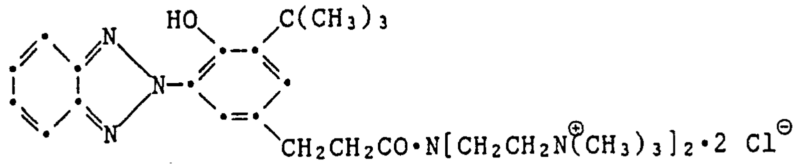

1,16 g [N,N-Bis-2-chloräthyl]-3-[3-(2-benztriazolyl)-4-hydroxy-5-t-butylphenyl]propionamid werden in 10 ml Toluol gelöst. 4,8 ml einer Lösung von Trimethylamin (4,2 M) in Aethanol und 0,1 ml DMF werden zugegeben und in einem Bombenrohr 24 Stunden bei 100 °C erhitzt. Danach lässt man abkühlen, wobei das Produkt ausfällt. Nach Waschen mit Diäthyläther und Trocknung im Vakuum erhält man 1,3 g (84 % der Theorie) des Dichlorids in Form weisser hygroskopischer Kristalle (Smp. 110 - 120 °C).

Anwendungsbeispiel 1:

Herstellung von Tintenstrahldruck-Beschichtungen

A. Beschichtung ohne Stabilisator:

16,4 g einer 10%-igen wässrigen Lösung von Polyvinylalkohol werden mit 0,03 g Di-t-octylphenolpolyethylenoxid Netzmittel, 0,3 g Polyfix®601 (Showa High Polymer Co.) Beizmittel gemischt und mit Wasser auf 27,0 g aufgefüllt.

Es werden 2,0 g Silika (Syloid® Typ 244, Grace & Co.) in die Lösung gerührt und mit Ultraschall dispergiert. Der pH der Dispersion wird mit 2N NaOH auf 7,0 eingestellt.

B. Beschichtung enthaltend einen Stabilisator:

Analog zu der Beschreibung unter A wird eine Dispersion zubereitet, jedoch wird das Polyfix®601 Beizmittel durch 0,6 g der Verbindung aus Beispiel 1b)

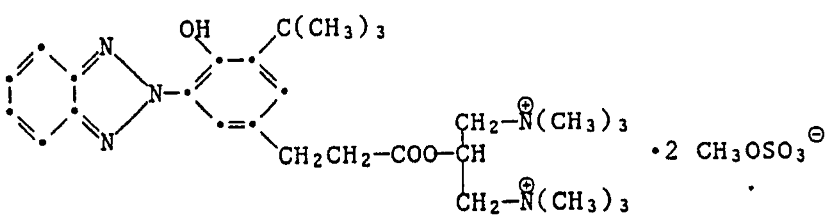

ersetzt. Die Gesamtmenge der wässrigen Phase beträgt 32,4 g.

Die erhaltenen Giessmischungen A und B werden mit einer Drahtspirale auf photographisches Papier in einer Dicke von 50 µm aufgetragen.

Die nach dem Trocknen erhaltenen Beschichtungen haben eine Trockenmasse von 7 g/m$^2$, wovon B I g/m$^2$ des Stabilisators enthält.

Die so präparierten Aufzeichnungsmaterialien werden jeweils mit einer purpur-roten Tinte in einer Tintenstrahldruckvorrichtung Typ "Think-Jet" (Hewlett-Packard) bedruckt. Die Tinten weisen folgende Zusammensetzung auf:

5 Teile C.I. Acid Red 35 bzw. C.I. Acid Red 249
50 Teile Diethylenglykol und
45 Teile Wasser.

Die Tinten werden durch einen Ultrafilter mit 0,3 µm Porenweite filtriert und in die Tintenpatrone des Gerätes "Think-Jet" gefüllt. Es werden bedruckte Proben mit einer Punktdichte von 192 x 96 Punkten pro Inch hergestellt.

Es wird nun die Farbdichte (Intensität) der bedruckten Proben mit einem Densitometer (Macbeth TR 924) unter Verwendung eines Status A-Filters bestimmt.

Prüfung der Lichtechtheit

Um die Lichtechtheit der bedruckten Proben zu testen, werden die Proben in einem Atlas Weatherometer mit einer Xenon-Lampe der Beleuchtungsstärke 81 klux hinter einem Filter aus 6 mm dickem Fensterglas bestrahlt.

Die Farbdichte wird erneut gemessen, um den prozentuellen Verlust an Farbdichte zu ermitteln.

Die Ergebnisse sind in der nachfolgenden Tabelle 1 zusammengefasst. Niedrigere Werte bedeuten höhere Lichtechtheit.

Tabelle 1

| Probe | FARBDICHTEVERLUST (%) | |
|---|---|---|
| | Acid Red 35 | Acid Red 249 |
| | 5 kJ/cm$^2$* | 15 kJ/m$^2$* |
| A (ohne Stabilisator) | 86 | 84 |
| B (mit Stabilisator) | 38 | 48 |

* Gemessene Menge an Strahlungsenergie im Bereich von 300-800 nm

Prüfung der Waschechtheit

Die bedruckte Probe mit der Beschichtung B wird in einem Trommelentwicklungsapparat (Jobo Autolab ATL-1) gewaschen. (Dieses Gerät wird üblicherweise zur Entwicklung photographischer Materialien eingesetzt).

Die bedruckte Probe wird im Trommelentwicklungsapparat jeweils 10mal für 30 Sekunden mit Wasser bei 25 °C gewaschen. Die Trommelgeschwindigkeit beträgt dabei das Minimum, um eine mechanische Beschädigung der Probenbeschichtung zur vermeiden.

Anschliessend wird die Probe getrocknet und ihre Farbdichte gemessen, um den prozentuellen Farbverlust beim Waschvorgang zu ermitteln. Die Ergebnisse sind nachfolgend zusammengefasst.

Tabelle 2

| Probe | FARBDICHTEVERLUST (%) | |
|---|---|---|
| | Acid Red 35 | Acid Red 249 |
| | 5 $kJ/cm^2$ | 15 $kJ/m^2$ |
| B (mit Stabilisator) | 0 | 0 |

Aus den erhaltenen Resultaten ist ersichtlich, dass die erfindungsgemässen Aufzeichnungsmaterialien, welche als Stabilisator eine Verbindung der Formel I enthalten, hervorragende Lichtechtheit bei gleichzeitiger Waschechtheit besitzen.

Anwendungsbeispiel 2:

Eine Lösung (A) des Farbstoffes C.I. Acid Yellow 23, welcher extrem schlechte Wasserbeständigkeit in Titenstrahldrucken aufweist, wird wie folgt hergestellt:
3 g C.I. Acid Yellow 23
30 g Diethylenglykol und
67 g Wasser.

Eine zweite Lösung (B) wird hergestellt aus:
3 g der Verbindung aus Beispiel Ib
30 g Diethylenglykol und
67 g Wasser.

Tinten für den Tintenstrahldruck können dadurch hergestellt werden, dass man entweder Lösung A zu Lösung B gibt oder umgekehrt. So können unter Rühren 3,1 g Lösung A zu 10 g Lösung B vor Einsetzen der Trübung zugegeben werden. In ähnlicher Weise können 10 g Lösung B zu 10 g Lösung A ohne Bildung eines Niederschlages zugegeben werden.

Die beiden Lösungsgemische werden durch ein Filter der Porenweite 0,3 µm filtriert und als Tinte für den Tintenstrahldruck analog Anwendungsbeispiel 1 verwendet.

Es werden ferner zwei Blindproben hergestellt. Sie bestehen aus Lösung A und Lösung C in den gleichen Mischverhältnissen, wie die Tinten aus Lösung A und Lösung B.

Dazu wird Lösung C wie folgt hergestellt:
3 g Lösung A
30 g Diethylenglykol und
67 g Wasser.

Als Tintenstrahldruckpapier wird ein Papier mit einer 5 $g/m^2$ Beschichtung bestehend aus 2,24 $g/m^2$ Polyvinylalkohol, 2,73 $g/m^2$ Silika und 0,03 $g/m^2$ Di-t-octylphenylpolyethylenoxid (8EO) verwendet. Dieses Papier wird mit den Tinten, wie in Anwendungsbeispiel 1 beschrieben ist, bedruckt und auf seine Waschechtheit geprüft. Die Ergebnisse sind in Tabelle 3 zusammengefasst. Niedrige Werte bedeuten höhere Waschechtheit.

Tabelle 3

| Probe | | | FARBDICHTEVERLUST |
|---|---|---|---|
| mit der Zusammensetzung | | | (%) |
| Teil A | Teil B | Teil C | Acid Yellow 23 |
| 10 | 10 | - | 92 |
| 10 | - | 10 | 96 |
| 3,1 | 10 | - | 84 |
| 3,1 | - | 10 | 90 |

Dieses Beispiel zeigt, dass die Verbindungen der Formel I direkt in die Tinte für den Tintenstrahldruck gegeben werden können, und dass diese Tinten Tintenstrahldrucken verbesserte Wasserbeständigkeit verleihen.

Anwendungsbeispiel 3:

Beschichtungsmassen für Tintenstrahldruckpapiere werden analog Anwendungsbeispiel 1 aus 16,4 g einer 10 %igen Polyvinylalkohol Lösung (Riedel de Haen), 0,015 g Invadin® JFC Netzmittel (Ciba-Geigy), 0,52 g eines erfindungsgemässen UV-Absorbers, 2,0 g Silika (Syloid®, Typ 244, W.R. Grace) und Wasser bis auf 33,2 g hergestellt. Der pH-Wert wird auf 7,0 mittels Lithiumhydroxid eingestellt. Die so erhaltene Masse trägt man mit einer 60 µm Rackelgiessmaschine auf polyäthylenbeschichtetes Papier auf und trocknet. Das Papier besitzt nun einen Auftrag von 8 g/m², wovon 1 g/m² auf den UV-Absorber entfällt.

Als Vergleich werden Papiere mit jeweils 0,52 g einer 10 %igen Beizmittellösung anstelle der 0,52 g UV-Absorber hergestellt. Dieses entspricht einem Beizmittelauftrag von ca. 0,1 g/m².

Als Beizmittel werden die Produkte Polyfix 601 (Showa High Polymer Co.), Merquat 100 (Chemviron), Tinofix EW (Ciba-Geigy AG) und Polyallylamine HCl (Nitto Boseki Col.) verwendet, welche beispielsweise in DE 3 640 359 und JP 61061 887 beschrieben sind.

Diese Papiere werden wie in Anwendungsbeispiel 1 beschrieben mit einer Tinte bedruckt, welche aus 4 g C.I. Acid Red 35 in einem Gemisch aus 48 g Wasser und 48 g Diäthylenglykol besteht (vgl. Anwendungsbeispiel 1). Dann werden die Licht- und Waschechtheiten wie in Anwendungsbeispiel 1 ermittelt. Die Ergebnisse sind in Tabelle 3 zusammengefasst.

Tabelle 3

| Probe | UV-Absorber/Beizmittel | Dichteverlust (%) | |
|---|---|---|---|
| | | nach 10 kJ/cm² Atlas Bestrahlung | im Waschechtheitstest |
| 1 | Verbindung nach Herstellungsbeispiel 1b | 46 | 0 |
| 2 | Verbindung nach Herstellungsbeispiel 2 | 43 | 0 |
| 3 | Verbindung nach Herstellungsbeispiel 8 | 38 | 0 |
| 4 | Verbindung nach Herstellungsbeispiel 10 | 48 | 0 |
| 5 | kein | 82 | 92 |
| 6 | Polyfix 601 | 81 | 88 |
| 7 | Polyallylamin HCl | 88 | 32 |
| 8 | Merquat 100 | 88 | 28 |
| 9 | Tinofix EW | 85 | 34 |

Anwendungsbeispiel 4

In diesem Beispiel wird gezeigt, dass auch kleinere Mengen von erfindungsgemässen UV-Absorbern in Tintenstrahldruckpapieren eine Verbesserung der Lichtechtheit bewirken.

Es werden Beschichtungsmassen aus jeweils 14,9 g einer 10 %igen Lösung von Polyvinylalkohol, 0.015 g Invadin® JFC Netzmittel, 0,24 g erfindungsgemässe UV-Absorber, 0,27 g Polyfix 601 Lösung (Showa High Polymer Co.), 2,0 g Silika und Wasser bis auf 30,5 g hergestellt. Die Beschichtungsmassen werden auf einem Papierträger aufgetragen. Nach dem Trocknen bedruckt man die so erhaltenen Papiere mit der Tintenstrahldrucktinte auf Basis Acid Red 35 gemäss Anwendungsbeispiel 3. Die Lichtechtheiten der bedruckten Proben werden dann wie in Anwendungsbeispiel 1 beschrieben getestet. Die Resultate sind in Tabelle 4 zusammengefasst.

Tabelle 4

| Probe | UV-Absorber | Farbdichteverlust (%) nach 10 kJ/cm$^2$ |
|---|---|---|
| 1 | kein | 78 |
| 2 | Verbindung nach Herstellungsbeispiel 1a* | 62 |
| 3 | Verbindung nach Herstellungsbeispiel 1b | 51 |
| 4 | Verbindung nach Herstellungsbeispiel 2 | 50 |
| 5 | Verbindung nach Herstellungsbeispiel 3b | 75 |
| 6 | Verbindung nach Herstellungsbeispiel 6 | 72 |
| 7 | Verbindung nach Herstellungsbeispiel 8 | 63 |
| 8 | Verbindung nach Herstellungsbeispiel 10 | 59 |

*Aus Gründen der Löslichkeit muss das Tintenstrahldruckpapier mit der Beschichtungsmasse bei pH 5,0 (mit $H_2SO_4$ eingestellt) beschichtet werden.

Anwendungsbeispiel 5:

Es wird ein wässriger Klarlack der folgenden Zusammensetzung hergestellt:

| | |
|---|---|
| Synthacryl® VSW 6484 (50 %) | 31,45 Teile |
| Maprenal® MF 915 (75 %) | 12,52 Teile |
| Maprenal® MF 927 (70 %) | 1,64 Teile |
| Additol® XW 329 | 0,04 Teile |
| deionisiertes Wasser | 20,00 Teile |
| Synthacryl® VSW 6483 (50 %) | 22,49 Teile |
| deionisiertes Wasser | 11,86 Teile |

1,5 % Stabilisator (bezogen auf Festkörper Lack) werden während 5 Minuten bei 3000 U/min in den Klarlack eingearbeitet. Der resultierende Lack wird mit deionisiertem Wasser auf Spritzfestigkeit eingestellt und auf ein vorbereitetes Substrat (coil coat-Blech mit Filter und wässrigem Silbermetallic-Basislack) gespritzt. Man brennt das Blech während 10 Minuten bei 80 °C und anschliessend während 30 Minuten bei 140 °C. Das so erhaltene Blech wird nach der Freibewitterung in Florida geprüft.

Als Vergleich dient ein entsprechender Lack ohne Stabilisator, mit dem auf die oben beschriebene Weise ein gleiches Blech beschichtet wurde. Tabelle 5 zeigt die Ergebnisse.

Tabelle 5

| Probe | UV-Absorber | % DOI*) |
|---|---|---|
| 1 | - | 49 |
| 2 | Verbindung **) | 85 |
| 3 | Verbindung nach Herstellungsbeispiel 8 | 89 |

*) Distinctness of Reflected Image (ASTM E 430) nach dreimonatiger Freibewitterung in Florida

**)

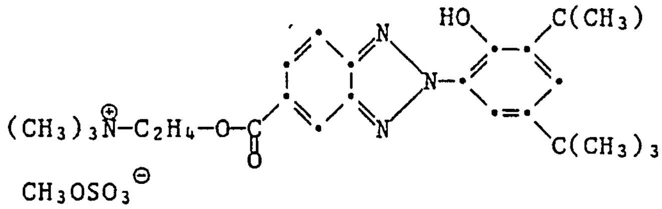

## Ansprüche

1. Aufzeichnungsmaterial enthaltend als Stabilisator mindestens eine verbindung der Formel I

$U\{SOL)_n$ (I),

worin n eine Zahl von 1 bis 4 bedeutet,
U ein Rest eines UV-Absorbers vom Typ Hydroxyphenyl-benztriazol, Zimtsäure oder Hydroxyphenyl-Triazin ist,
SOL, gleich oder verschieden, eine Gruppe der Formel Ia

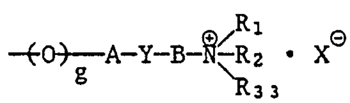

(Ia)

bedeutet, worin
g für 0 oder 1 steht,
A eine direkte Bindung, $C_1$-$C_6$-Alkylen oder -Alkyliden, oder eine Gruppe der Formel Ib

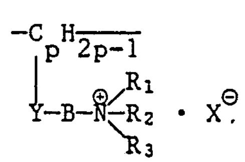

(Ib)

bedeutet, wobei p eine Zahl von 1 bis 6 ist,
Y eine direkte Bindung oder eine der folgenden Gruppen ist:
-CO-, -COO-, -OOC-, -CO-N(R′)-, -(R′)N-CO-, $-SO_2-N(R')-$, $-(R')N-SO_2-$,

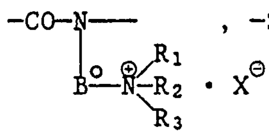, 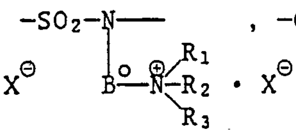, 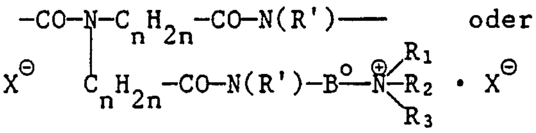 oder

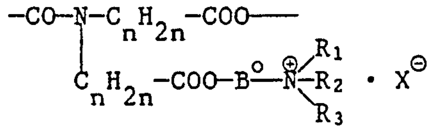,

B eine direkte Bindung bedeutet oder ein gegebenenfalls mit OH substituiertes $C_2$-$C_6$-Alkylen oder -Alkyliden ist, welches gegebenenfalls durch ein -O- oder durch ein oder zwei - $\overset{\oplus}{N}X^{\ominus}(R')_2$- unterbrochen ist, oder eine Gruppe der Formel Ic

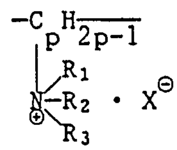 oder 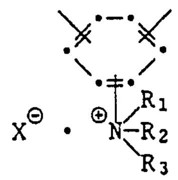

(Ic) (IC)

ist, oder die Gruppe

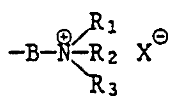

für einen gesättigten oder ungesättigten 1 bis 3-kernigen N-Heterocyklus steht, der 1-4 N-Atome als Ringglieder enthält, von denen mindestens eines quaterniert ist, unter den Bedingungen, dass

a) im Falle g = 1, A, Y und B nicht gleichzeitig eine direkte Bindung bedeuten;

b) im Falle g = 1 und A = direkte Bindung, Y -CO- oder eine direkte Bindung bedeutet; und

c) im Falle B = eine direkte Bindung, auch Y eine direkte Bindung ist;

R′ Wasserstoff, $C_1$-$C_4$-Alkyl oder $C_2$-$C_3$-Hydroxyalkyl ist, B° $-(CH_2)_m-$ oder eine der folgenden Gruppen bedeutet:

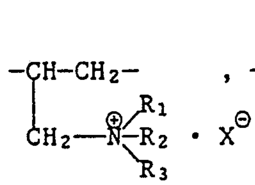, 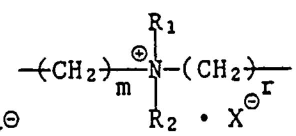 oder 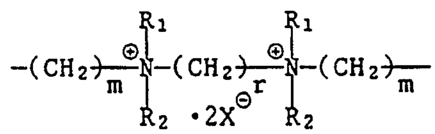,

wobei m und r unabhängig voneinander 2 oder 3 bedeuten,

$R_{33}$ eine Bedeutung von $R_3$ hat oder für eine Gruppe der Formeln Id und Ie

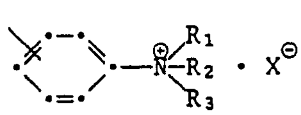 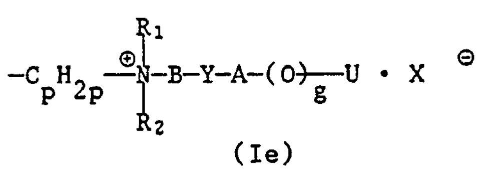

(Id) (Ie)

steht, worin g, p, B, Y, A und U die zuvor genannte Bedeutung haben,

$R_1$, $R_2$ und $R_3$ unabhängig voneinander Wasserstoff, $C_1$-$C_8$-Alkyl, durch ein -COOR″ oder 1 bis 3 OH substituiertes $C_1$-$C_8$-Alkyl, durch ein oder mehrere -O- unterbrochenes $C_2$-$C_8$-Hydroxyalkyl, mit einem OH

substituiertes -($C_1$-$C_8$)Alkylen-$COO^{\ominus}$, -($C_1$-$C_8$)Alkyliden-$COO^{\ominus}$, -($C_2$-$C_8$)Alkylen-$SO_3^{\ominus}$ oder -($C_2$-$C_8$)Alkyliden-$SO_3^{\ominus}$, $C_3$-$C_5$-Alkenyl, $C_5$-$C_7$-Cycloalkyl, Phenyl, Tolyl, Benzyl oder Glycidyl bedeuten, oder $R_1$ zusammen mit $R_2$ und gegebenenfalls mit $R_3$ sowie zusammen mit dem $\overset{\oplus}{N}$-Atom, an das sie gebunden sind, einen N-Heterocyklus bilden, der gegebenenfalls 1-3 N-Atome oder ein O-Atom als Ringglieder enthält,
$R''$ Wasserstoff oder $C_1$-$C_4$-Alkyl ist, und
$X^{\ominus}$, falls nicht in $R_1$, $R_2$, $R_3$ oder $R_{33}$ vorhanden, ein farbloses organisches oder anorganisches Anion bedeutet.

2. Aufzeichnungsmaterial gemäss Anspruch 1 von Verbindungen der Formel I, worin in der Formel Ia g = 0 ist.

3. Aufzeichnungsmaterial gemäss Anspruch 2, worin $R_{33}$ in der Formel Ia eine Bedeutung von $R_3$ hat.

4. Aufzeichnungsmaterial gemäss Anspruch 3, worin die Formel Ia einer Gruppe der Formel If

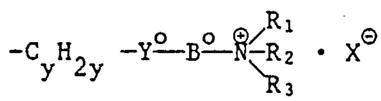

(If)

Y° -COO-, -CONH- oder

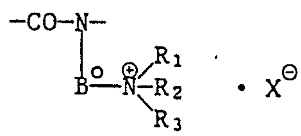

bedeutet, y eine Zahl von 0 bis 6 ist, und p, B°, $R_1$, $R_2$ und $R_3$ die in Anspruch 1 genannte Bedeutung haben.

5. Aufzeichnungsmaterial gemäss Anspruch 1, worin die Verbindung der Formel I einer Verbindung der Formel II oder III

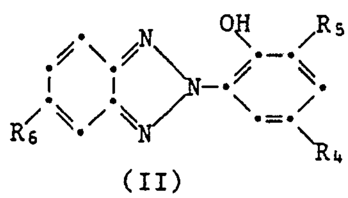

(II)

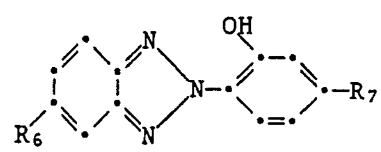

(III)

entspricht, worin
$R_4$ Halogen, $C_1$-$C_4$-Alkoxy, $C_1$-$C_8$-Alkyl, $C_5$-$C_6$-Cycloalkyl, Phenyl, Phenyl-$C_1$-$C_4$-Alkyl oder eine Gruppe der Formel Ia ist,
$R_5$ und $R_6$ unabhängig voneinander Wasserstoff sind, oder eine Bedeutung von $R_4$ haben,
$R_7$ OH, $C_1$-$C_8$-Alkoxy, welches durch 1 bis 3 OH oder 1 bis 2 -COOH substituiert sein kann, $C_1$-$C_8$-Alkanoyloxy, eine Gruppe der Formel Ia mit g = 1 oder Glycidyloxy ist,
unter der Bedingung, dass mindestens eines von $R_4$, $R_5$ und $R_6$ in der Formel II und mindestens eines von $R_6$ und $R_7$ in der Formel III für eine Gruppe der Formel Ia steht.

6. Aufzeichnungsmaterial gemäss Anspruch 5, worin $R_4$ Halogen, $C_1$-$C_5$-Alkyl, Cyclohexyl oder eine Gruppe der Formel Ia ist, $R_5$ Wasserstoff ist oder eine Bedeutung von $R_4$ hat, $R_6$ für Wasserstoff, Halogen oder eine Gruppe der Formel Ia steht, und $R_7$ OH, gegebenenfalls mit 1 bis 3 OH substituiertes $C_1$-$C_4$-Alkoxy, Glycidyloxy, $C_2$-$C_3$-Alkanoyloxy oder eine Gruppe der Formel Ia mit g = 1 bedeutet.

7. Aufzeichnungsmaterial gemäss Anspruch 6, worin in der Formel II $R_4$ $C_1$-$C_4$-Alkyl oder eine Gruppe der Formel If bedeutet,
$R_5$ Wasserstoff, $C_1$-$C_4$-Alkyl oder eine Gruppe If ist, und $R_6$ Wasserstoff, Cl oder eine Gruppe If bedeutet, worin y für 0 steht, unter der Bedingung, dass mindestens eines von $R_4$, $R_5$ und $R_6$ eine Gruppe der Formel If darstellt.

8. Aufzeichnungsmaterial gemäss Anspruch 1, worin die Verbindung der Formel I einer Verbindung der Formel V

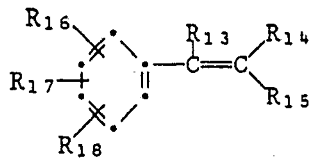

(V)

entspricht, worin

$R_{13}$ Wasserstoff, $C_1$-$C_8$-Alkyl, $C_3$-$C_8$-Alkenyl, Phenyl-$C_1$-$C_4$-Alkyl oder eine Gruppe der Formel

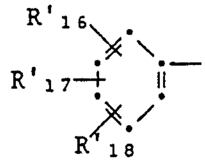

bedeutet, $R_{14}$ und $R_{15}$ unabhängig voneinander -C≡N, -CO-$C_1$-$C_4$-Alkyl, -COO-$R_{19}$ oder -CO-$NR_{20}R_{21}$ bedeuten, $R_{16}$, $R_{17}$, $R_{18}$, $R'_{16}$, $R'_{17}$ und $R'_{18}$ unabhängig voneinander für Wasserstoff, $C_1$-$C_4$-Alkyl, Phenyl, Glycidyl, OH, Halogen, gegebenenfalls mit 1 bis 3 OH substituiertes $C_1$-$C_4$-Alkoxy, Phenoxy, Benzyloxy oder eine Gruppe der Formel Ia stehen, und

$R_{19}$, $R_{20}$ und $R_{21}$ unabhängig voneinander Wasserstoff, $C_1$-$C_4$-Alkyl, welches gegebenenfalls mit 1 bis 3 OH substituiert ist, $C_5$-$C_7$-Cycloalkyl, Phenyl, Benzyl, Tolyl, Glycidyl oder eine Gruppe der Formel Ig

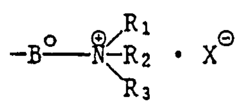

(Ig)

bedeuten, worin $B^o$, $R_1$, $R_2$ und $R_3$ die in Anspruch 1 genannte Bedeutung haben,

unter der Bedingung, dass mindestens eines von $R_{16}$, $R_{17}$, $R_{18}$, $R'_{16}$, $R'_{17}$ oder $R'_{18}$ eine Gruppe der Formel Ia oder mindestens eines von $R_{19}$, $R_{20}$ oder $R_{21}$ eine Gruppe der Formel Ig bedeuten.

9. Aufzeichnungsmaterial gemäss Anspruch 8, worin die Verbindung der Formel V einer Verbindung der Formel Va

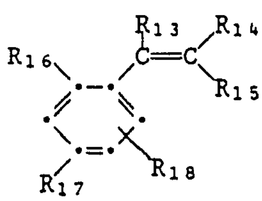

(Va)

entspricht, worin

$R_{13}$ Wasserstoff, $C_1$-$C_4$-Alkyl oder eine Gruppe

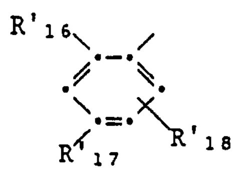

darstellt, und $R_{14}$, $R_{15}$, $R_{16}$, $R_{17}$, $R_{18}$, $R'_{16}$, $R'_{17}$ und $R'_{18}$ die in Anspruch 10 genannte Bedeutung haben.

10. Aufzeichnungsmaterial gemäss Anspruch 1, worin die Verbindung der Formel I einer Verbindung der Formel VI

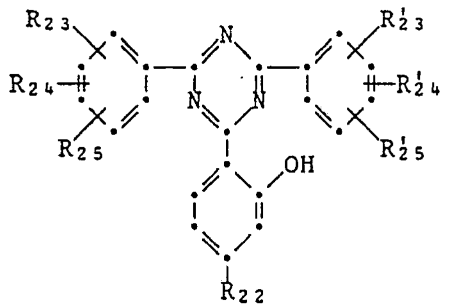

(VI)

entspricht, worin

$R_{22}$ eine Gruppe der Formel Ia mit g = 1 ist und $R_{23}$, $R_{24}$, $R_{25}$, $R'_{23}$, $R'_{24}$ und $R'_{25}$ für Wasserstoff, OH, Halogen, $C_1$-$C_4$-Alkoxy, $C_1$-$C_4$-Alkyl, Phenyl oder eine Gruppe der Formel Ia mit g = 1 stehen.

11. Aufzeichnungsmaterial gemäss Anspruch 10, worin die Verbindung der Formel VI einer Verbindung der Formel VIa

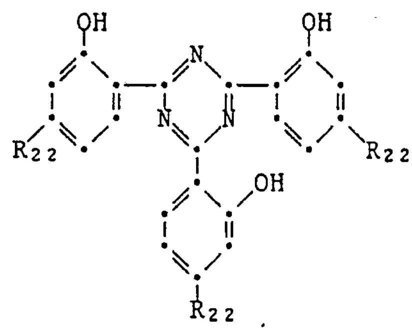

(VIa)

entspricht, und $R_{22}$ für eine Gruppe der Formel Ia mit g = 1 steht.

12. Aufzeichnungsmaterial gemäss Anspruch 1, worin $X^\ominus$ in der Formel Ia ist Chlorid oder Bromid, $C_1$-$C_4$-Alkyl-$COO^\ominus$, $C_1$-$C_4$-Alkyl-$OSO_3^\ominus$ oder $R^o$-$SO_3^\ominus$ bedeutet, wobei $R^o$ für Methyl, Tolyl oder -$CF_3$ steht.

13. Aufzeichnungsmaterial gemäss Anspruch 1, dadurch gekennzeichnet, dass das Aufzeichnungsmaterial für Tintenstrahldruck geeignet ist.

14. Aufzeichnungsmaterial gemäss Anspruch 13, dadurch gekennzeichnet, dass es aus einem Trägermaterial mit einer durch Tintenstrahl bedruckbaren Oberfläche besteht.

15. Aufzeichnungsmaterial gemäss Anspruch 14, dadurch gekennzeichnet, dass es ein beschichtetes Papier ist.

16. Verfahren zur Herstellung eines gegen Lichtschädigung stabilisierten Aufzeichnungsmaterials für den Tintenstrahldruck, dadurch gekennzeichnet, dass man einen zweidimensionalen Träger mit einer Beschichtungsmasse beschichtet, welche mindestens eine Verbindung der Formel I gemäss Anspruch 1 enthält.

17. Verwendung von Verbindungen der Formel I gemäss Anspruch 1 als Lichtschutzmittel für Aufzeichnungsmaterialien.

18. Verbindung der Formel I°

$U\{SOL^o)_{n'}$ (I°),

worin n' eine Zahl von 1 bis 4 bedeutet,

U ein Rest eines UV-Absorbers vom Typ Hydroxyphenyl-benztriazol, Zimtsäure oder Triazin ist,

SOL°, gleich oder verschieden, eine Gruppe der Formel I°a

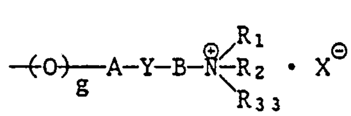

(I°a)

bedeutet, worin

g für 0 oder 1 steht,

A eine direkte Bindung, $C_1$-$C_6$-Alkylen oder eine Gruppe der Formel I°b

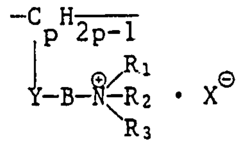

(I°b)

bedeutet, wobei p eine Zahl von 1 bis 6 ist,
Y eine direkte Bindung oder eine der folgenden Gruppen ist:
-CO-, -COO-, -OOC-, -CO-N(R′)-, -(R′)N-CO-, $-SO_2-N(R')-$, $-(R')N-SO_2-$,

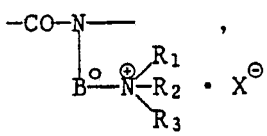 , 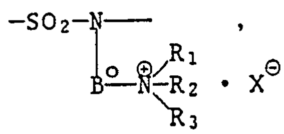 , 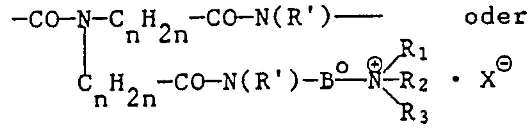 oder

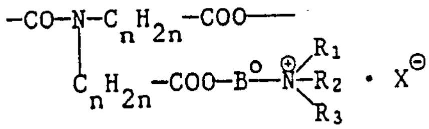 ,

B eine direkte Bindung bedeutet oder ein gegebenenfalls mit OH substituiertes $C_2$-$C_6$-Alkylen ist, welches gegebenenfalls durch ein -O- oder durch ein oder zwei - $\overset{\oplus}{N}X^{\ominus}(R')_2$- unterbrochen ist, oder eine Gruppe der Formeln I°c oder I°C

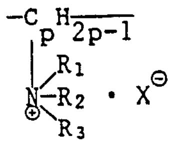 oder 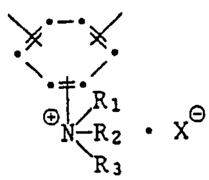

(I°c) (I°C)

ist, oder die Gruppe

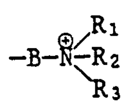

für einen gesättigten oder ungesättigten 1 bis 3-kernigen N-Heterocyklus steht, der 1-4 N-Atome als Ringglieder enthält, von denen mindestens eines quaterniert ist, unter den Bedingungen, dass
a) im Falle g = 1, A, Y und B nicht gleichzeitig eine direkte Bindung bedeuten;
b) im Falle g = 1 und A = direkte Bindung, Y -CO- oder eine direkte Bindung bedeutet; und
c) im Falle B = eine direkte Bindung, auch Y eine direkte Bindung ist;
R′ Wasserstoff, $C_1$-$C_4$-Alkyl oder $C_2$-$C_3$-Hydroxyalkyl ist,
B° $-(CH_2)_m-$ oder eine der folgenden Gruppen bedeutet:

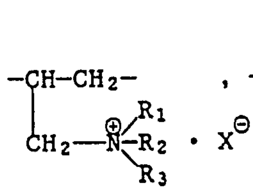 , 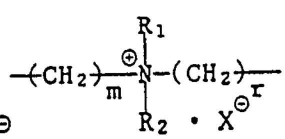 oder 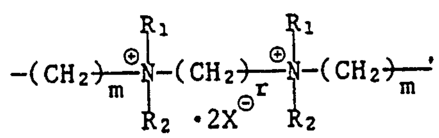 ,

wobei m und r unabhängig voneinander 2 oder 3 bedeuten,
$R_{33}$ eine Bedeutung von $R_3$ hat oder für eine Gruppe der Formeln I°d und I°e

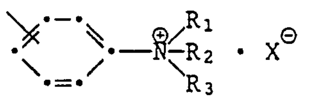
(I°d)

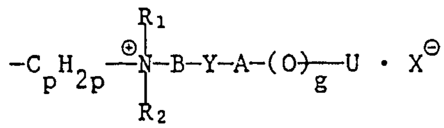
(I°e)

steht, worin g, p, B, Y, A und U die zuvor genannte Bedeutung haben, $R_1$, $R_2$ und $R_3$ unabhängig voneinander $C_1$-$C_8$-Alkyl oder durch ein -COOR″ oder 1 bis 3 OH substituiertes $C_1$-$C_8$-Alkyl, durch ein oder mehrere -O- unterbrochenes $C_2$-$C_8$-Hydroxyalkyl, mit einem OH substituiertes -($C_1$-$C_8$)Alkylen-$COO^{\ominus}$, -($C_1$-$C_8$)Alkyliden-$COO^{\ominus}$, -($C_2$-$C_8$)Alkylen-$SO_3^{\ominus}$ oder -($C_2$-$C_8$)Alkyliden-$SO_3^{\ominus}$, $C_3$-$C_5$-Alkenyl, $C_5$-$C_7$-Cycloalkyl, Phenyl, Tolyl, Benzyl oder Glycidyl bedeuten, oder $R_1$ zusammen mit $R_2$ und gegebenenfalls mit $R_3$ sowie zusammen mit dem $\overset{\oplus}{N}$-Atom, an das sie gebunden sind, einen N-Heterocyklus bilden, der gegebenenfalls 1-3 N-Atome oder ein O-Atom als Ringglieder enthält,
R″ Wasserstoff oder $C_1$-$C_4$-Alkyl ist, und
$X^{\ominus}$, falls nicht in $R_1$, $R_2$, $R_3$ oder $R_{33}$ vorhanden, ein farbloses organisches oder anorganisches Anion bedeutet,
unter den Bedingungen, dass

A) im Falle U für einen Rest vom Typ Hydroxyphenyl-Benztriazol steht und n in der Formel I° 1 bedeutet, Y in der Formel I° die Bedeutung von $Y_1$ hat, wobei
$Y_1$ eine der folgenden Gruppen ist

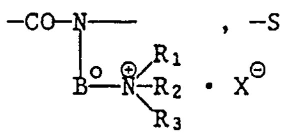 , 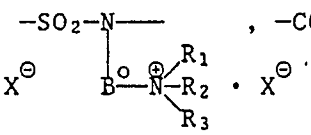 , 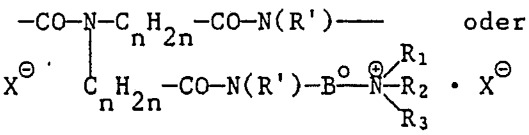 oder

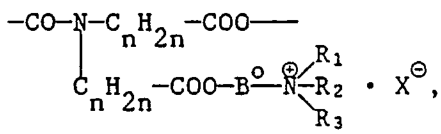,

B) im Falle U für einen Rest vom Typ Hydroxybenzophenon steht und n in der Formel I°a 1 oder 2 bedeutet, g = 0 ist;

C) im Falle U für einen Zimtsäurerest mit 1 oder 2 -C≡N-Gruppen steht, n in der Formel I° 3 oder 4 ist; oder

D) im Falle U für einen Hydroxyphenyl-Triazinrest steht und in der Formel I°a g = 1, A = Alkylen und Y eine direkte Bindung bedeuten, B nicht für eine direkte Bindung oder Alkylen stehen kann, oder wenn g = 1 und A eine direkte Bindung bedeuten, Y nicht -CO- sein kann.

19. Verbindung gemäss Anspruch 18, worin die Verbindung der Formel I° einer Verbindung der Formel II° oder III°

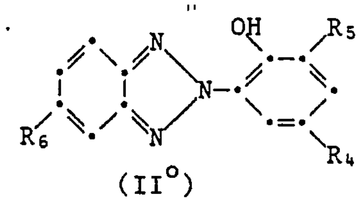
(II°)

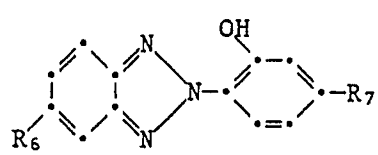
(III°)

entspricht, worin
$R_4$ Halogen, $C_1$-$C_4$-Alkoxy, $C_1$-$C_8$-Alkyl, $C_5$-$C_6$-Cycloalkyl, Phenyl, Phenyl-$C_1$-$C_4$-Alkyl oder eine Gruppe der Formel I°a ist,
$R_5$ und $R_6$ unabhängig voneinander Wasserstoff sind, oder eine Bedeutung von $R_4$ haben,

$R_7$ OH, $C_1$-$C_8$-Alkoxy, welches durch 1 bis 3 OH oder 1 bis 2 -COOH substituiert sein kann, $C_1$-$C_8$-Alkanoyloxy, eine Gruppe der Formel Ia mit g = 1 oder Glycidyloxy ist,

unter der Bedingung, dass mindestens eines von $R_4$, $R_5$ und $R_6$ in der Formel II° und mindestens eines von $R_6$ und $R_7$ in der Formel III° für eine Gruppe der Formel I°a steht, und in der Formel I°a falls g = 0 und A = Alkylen bedeuten, Y nicht eine direkte Bindung, -COO- oder -CONHsein kann, oder

falls g = 1, A = Alkylen und Y eine direkte Bindung bedeuten, B nicht für eine direkte Bindung oder Alkylen stehen kann.

20. Verbindung gemäss Anspruch 19, worin $R_4$ Halogen, $C_1$-$C_5$-Alkyl, Cyclohexyl oder eine Gruppe der Formel I°a ist, $R_5$ Wasserstoff ist, oder eine Bedeutung von $R_4$ hat, $R_6$ für Wasserstoff, Halogen oder eine Gruppe der Formel I°a steht, und $R_7$ OH, gegebenenfalls mit 1 bis 3 OH oder mit 1 bis 2 COOH substituiertes $C_1$-$C_4$-Alkoxy, Glycidyloxy, $C_2$-$C_3$-Alkanoyloxy oder eine Gruppe der Formel I° a mit g = 1 bedeutet.

21. Verbindung gemäss Anspruch 20, worin in der Formel II° $R_4$ $C_1$-$C_4$-Alkyl oder eine Gruppe der Formel I°f

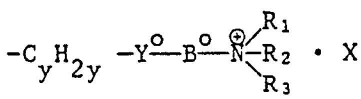

(I°f)

bedeutet,

$R_5$ Wasserstoff, $C_1$-$C_4$-Alkyl oder eine Gruppe I°f ist, und $R_6$ Wasserstoff, Cl oder eine Gruppe I°f bedeutet, worin y für 0 steht, unter der Bedingung, dass mindestens eines von $R_4$, $R_5$ und $R_6$ eine Gruppe der Formel I°f darstellt.

22. Verbindung gemäss Anspruch 18, worin die Verbindung der Formel I° einer Verbindung der Formel V°

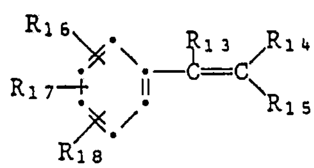

(V°)

entspricht, worin

$R_{13}$ Wasserstoff, $C_1$-$C_8$-Alkyl, $C_3$-$C_8$-Alkenyl, Phenyl-$C_1$-$C_4$-Alkyl oder eine Gruppe der Formel

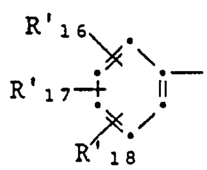

bedeutet, $R_{14}$ und $R_{15}$ unabhängig voneinander -C≡N, -CO-$C_1$-$C_4$-Alkyl, -COO-$R_{19}$ oder -CO-$NR_{20}R_{21}$ bedeuten, $R_{16}$, $R_{17}$, $R_{18}$, $R'_{16}$, $R'_{17}$ und $R'_{18}$ unabhängig voneinander für Wasserstoff, $C_1$-$C_4$-Alkyl, Phenyl, Glycidyl, OH, Halogen, gegebenenfalls mit 1 bis 3 OH substituiertes $C_1$-$C_4$-Alkoxy, Phenoxy, Benzyloxy oder eine Gruppe der Formel I°a stehen, und

$R_{19}$, $R_{20}$ oder $R_{21}$ unabhängig voneinander Wasserstoff, $C_1$-$C_4$-Alkyl, welches gegebenenfalls mit 1 bis 3 OH substituiert ist, $C_5$-$C_7$-Cycloalkyl, Phenyl, Benzyl, Tolyl, Glycidyl oder eine Gruppe der Formel I°g

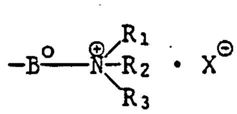

(I°g)

bedeuten, worin B°, $R_1$, $R_2$ und $R_3$ die in Anspruch 20 genannte Bedeutung haben,

unter den Bedingungen, dass mindestens eines von $R_{16}$, $R_{17}$, $R_{18}$, $R'_{16}$, $R'_{17}$ und $R'_{18}$ eine Gruppe der

Formel I°a oder mindestens eines von $R_{19}$, $R_{20}$ oder $R_{21}$ eine Gruppe der Formel I°g bedeuten, und falls $R_{14}$ und/oder $R_{15}$ für -C≡N stehen, mindestens 3 Gruppen der Formel I°a und/oder I°g vorhanden sind.

23. Verbindung gemäss Anspruch 22, worin die Verbindung der Formel V° einer Verbindung der Formel V°a

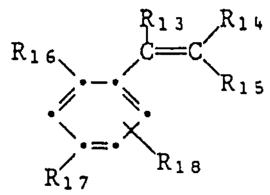

(V°a)

entspricht, worin

$R_{13}$ Wasserstoff, $C_1$-$C_4$-Alkyl oder eine Gruppe

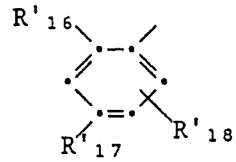

darstellt, und $R_{14}$, $R_{15}$, $R_{16}$, $R_{17}$, $R_{18}$, $R'_{16}$, $R'_{17}$ und $R'_{18}$ die in Anspruch 22 genannte Bedeutung haben.

24. Verbindung gemäss Anspruch 18, worin die Verbindung der Formel I° einer Verbindung der Formel VI°

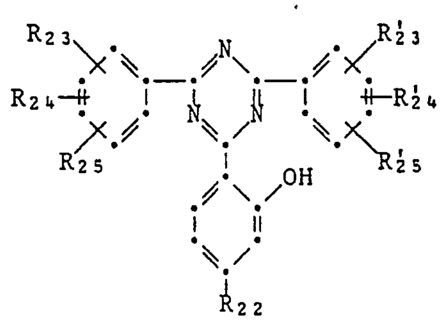

(VI°)

entspricht, worin

$R_{22}$ eine Gruppe der Formel I°a mit g = 1 ist und $R_{23}$, $R_{24}$, $R_{25}$, $R'_{23}$, $R'_{24}$ und $R'_{25}$ für Wasserstoff, OH, Halogen, $C_1$-$C_4$-Alkoxy, $C_1$-$C_4$-Alkyl, Phenyl oder eine Gruppe der Formel I°a mit g = 1 stehen, unter der Bedingung, dass falls in der Formel I°a g = 1, A = Alkylen und Y eine direkte Bindung bedeuten, B nicht für eine direkte Bindung oder Alkylen steht, oder

falls g = 1 und A eine direkte Bindung bedeuten, Y nicht -CO- sein kann.

25. Verbindung gemäss Anspruch 24, worin die Verbindung der Formel VI° einer Verbindung der Formel VI°a

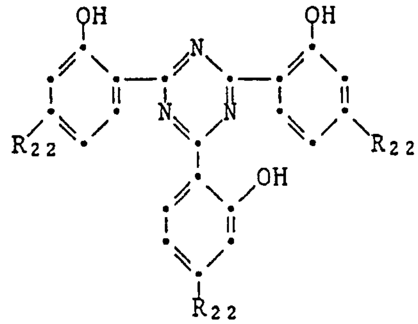

(VI°a)

entspricht, und $R_{22}$ für eine Gruppe der Formel I°a mit g = 1 steht.

26. Verbindungen gemäss Anspruch 18, worin $X^{\ominus}$ in der Formel Ia Halogen$^{\ominus}$, $C_1$-$C_4$-Alkyl-$COO^{\ominus}$, $C_1$-$C_4$-Alkyl-$OSO_3^{\ominus}$ oder $R^o$-$SO_3^{\ominus}$ bedeutet, wobei $R^o$ für Methyl, Tolyl oder -$CF_3$ steht.

27. Verwendung von Verbindungen der Formel I° gemäss Anspruch 18 als Stabilisator für organische Materialien.

28. Verwendung gemäss Anspruch 27 als Stabilisator für Aufzeichnungsmaterialien für den Tintenstrahldruck.

29. Verwendung gemäss Anspruch 27 als Stabilisator für Tinten für den Tintenstrahldruck.

30. Zusammensetzung enthaltend ein organisches Material und mindestens eine Verbindung der Formel I° gemäss Anspruch 18 als Stabilisator.

31. Zusammensetzung gemäss Anspruch 30, dadurch gekennzeichnet, dass das organische Material ein synthetisches Polymer ist.

32. Zusammensetzung gemäss Anspruch 30, dadurch gekennzeichnet, dass das organische Material ein Lack ist.

33. Zusammensetzung gemäss Anspruch 30, dadurch gekennzeichnet, dass das organische Material eine Tinte ist.

34. Tinte enthaltend als Stabilisator mindestens eine Verbindung der Formel I

$U\text{-}(SOL)_n$ (I),

worin n eine Zahl von 1 bis 4 bedeutet,

U ein Rest eines UV-Absorbers vom Typ Hydroxyphenyl-benztriazol, Zimtsäure oder Hydroxyphenyl-Triazin ist,

SOL, gleich oder verschieden, eine Gruppe der Formel Ia

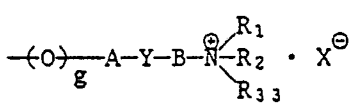

(Ia)

bedeutet, worin

g für 0 oder 1 steht,

A eine direkte Bindung, $C_1$-$C_6$-Alkylen oder -Alkyliden, oder eine Gruppe der Formel Ib

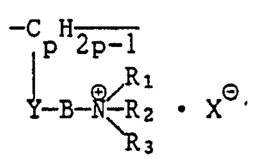

(Ib)

bedeutet, wobei p eine Zahl von 1 bis 6 ist,

Y eine direkte Bindung oder eine der folgenden Gruppen ist:

-CO-, -COO-, -OOC-, -CO-N(R')-, -(R')N-CO-, $-SO_2-N(R')-$, $-(R')N-SO_2-$,

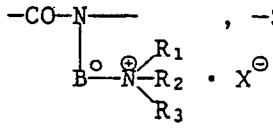 , 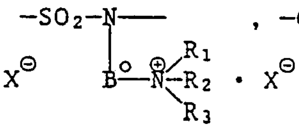 , 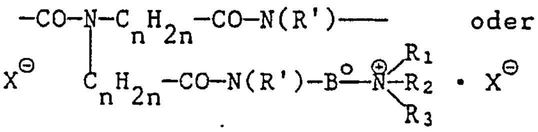 oder

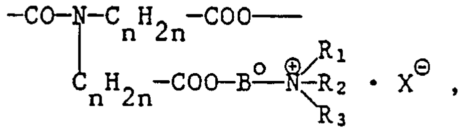 ,

B eine direkte Bindung bedeutet oder ein gegebenenfalls mit OH substituiertes $C_2$-$C_6$-Alkylen oder -Alkyliden ist, welches gegebenenfalls durch ein -O- oder durch ein oder zwei -$\overset{\oplus}{N}X^{\ominus}(R')_2$- unterbrochen ist, oder eine Gruppe der Formel Ic

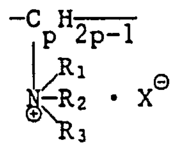 oder 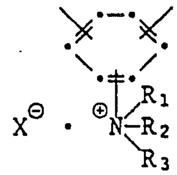

(Ic) (IC)

ist, oder die Gruppe

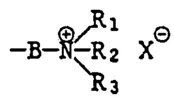

für einen gesättigten oder ungesättigten 1 bis 3-kernigen N-Heterocyklus steht, der 1-4 N-Atome als Ringglieder enthält, von denen mindestens eines quaterniert ist, unter den Bedingungen, dass
a) im Falle g = 1, A, Y und B nicht gleichzeitig eine direkte Bindung bedeuten;
b) im Falle g = 1 und A = direkte Bindung, Y -CO- oder eine direkte Bindung bedeutet; und
c) im Falle B = eine direkte Bindung, auch Y eine direkte Bindung ist;
R′ Wasserstoff, $C_1$-$C_4$-Alkyl oder $C_2$-$C_3$-Hydroxyalkyl ist,
B° $-(CH_2)_m-$ oder eine der folgenden Gruppen bedeutet:

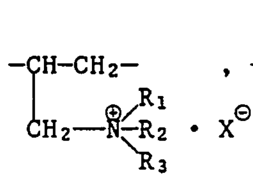 , 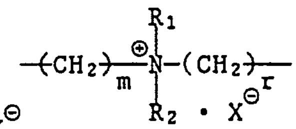 oder 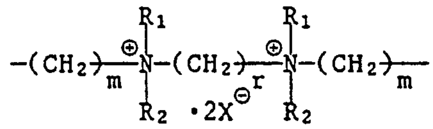 ,

wobei m und r unabhängig voneinander 2 oder 3 bedeuten, $R_{33}$ eine Bedeutung von $R_3$ hat oder für eine Gruppe der Formeln Id und Ie

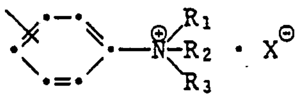 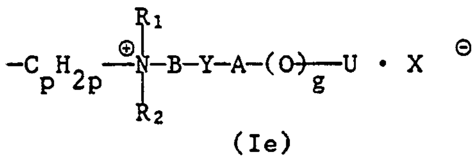

(Id) (Ie)

steht, worin g, p, B, Y, A und U die zuvor genannte Bedeutung haben,
$R_1$, $R_2$ und $R_3$ unabhängig voneinander $C_1$-$C_8$-Alkyl, durch ein -COOR″ der 1 bis 3 OH substituiertes $C_1$-$C_8$-Alkyl, durch ein oder mehrere -O- unterbrochenes $C_2$-$C_8$-Hydroxyalkyl, mit einem OH substituiertes --

$(C_1-C_8)$-Alkylen-$COO^{\ominus}$, -$(C_1-C_8)$Alkyliden-$COO^{\ominus}$, -$(C_2-C_8)$Alkylen-$SO_3^{\ominus}$ oder -$(C_2-C_8)$Alkyliden-$SO_3^{\ominus}$, $C_3$-$C_5$-Alkenyl, $C_5$-$C_7$-Cycloalkyl, Phenyl, Tolyl, Benzyl oder Glycidyl bedeuten, oder $R_1$ zusammen mit $R_2$ und gegebenenfalls mit $R_3$ sowie zusammen mit dem $\overset{\oplus}{N}$-Atom, an das sie gebunden sind, einen N-Heterocyklus bilden, der gegebenenfalls 1-3 N-Atome oder ein O-Atom als Ringglieder enthält,
$R''$ Wasserstoff oder $C_1$-$C_4$-Alkyl ist, und
$X^{\ominus}$, falls nicht in $R_1$, $R_2$, $R_3$ oder $R_{33}$ vorhanden, ein farbloses organisches oder anorganisches Anion bedeutet.

35. Tinte gemäss Anspruch 34, dadurch gekennzeichnet, dass die Tinte für den Tintenstrahldruck verwendet wird.

36. Tinte gemäss Anspruch 34, dadurch gekennzeichnet, dass die Tinte Wasser enthält.

37. Tinte gemäss Anspruch 34, dadurch gekennzeichnet, dass diese den Stabilisator der Formel I in einer Konzentration von 0,01 bis 20 Gew.-%, insbesondere von 0,5 bis 10 Gew.-%, enthält.

38. Tinte gemäss Anspruch 37, dadurch gekennzeichnet, dass diese zusätzlich mindestens einen wasserlöslichen Azofarbstoff enthält.

Patentansprüche für folgenden Vertragsstaat: ES

1. Aufzeichnungsmaterial enthaltend als Stabilisator mindestens eine Verbindung der Formel I
$U\{SOL)_n$ (I),
worin n eine Zahl von 1 bis 4 bedeutet,
U ein Rest eines UV-Absorbers vom Typ Hydroxyphenyl-benztriazol, Zimtsäure oder Hydroxyphenyl-Triazin ist,
SOL, gleich oder verschieden, eine Gruppe der Formel Ia

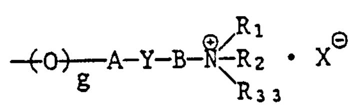
(Ia)

bedeutet, worin
g für 0 oder 1 steht,
A eine direkte Bindung, $C_1$-$C_6$-Alkylen oder -Alkyliden, oder eine Gruppe der Formel Ib

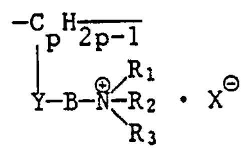
(Ib)

bedeutet, wobei p eine Zahl von 1 bis 6 ist,
Y eine direkte Bindung oder eine der folgenden Gruppen ist:
-CO-, -COO-, -OOC-, -CO-N(R')-, -(R')N-CO-, $-SO_2-N(R')-$, $-(R')N-SO_2-$,

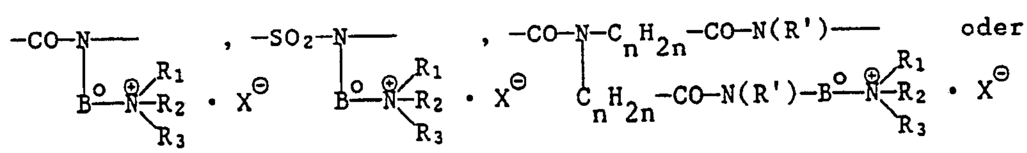
, , oder

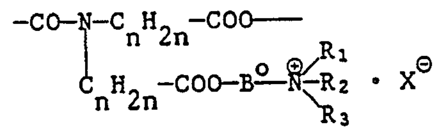
,

B eine direkte Bindung bedeutet oder ein gegebenenfalls mit OH substituiertes $C_2$-$C_6$-Alkylen oder -Alkyliden ist, welches gegebenenfalls durch ein -O- oder durch ein oder zwei - $\overset{\oplus}{N}X^{\ominus}(R')_2$- unterbrochen ist, oder eine Gruppe der Formel Ic

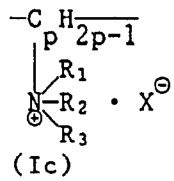

(Ic)

oder

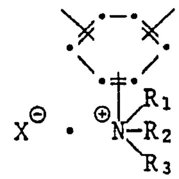

(IC)

ist, oder die Gruppe

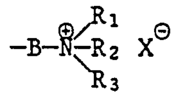

für einen gesättigten oder ungesättigten 1 bis 3-kernigen N-Heterocyklus steht, der 1-4 N-Atome als Ringglieder enthält, von denen mindestens eines quaterniert ist, unter den Bedingungen, dass

a) im Falle g = 1, A, Y und B nicht gleichzeitig eine direkte Bindung bedeuten;

b) im Falle g = 1 und A = direkte Bindung, Y -CO- oder eine direkte Bindung bedeutet; und

c) im Falle B = eine direkte Bindung, auch Y eine direkte Bindung ist;

R′ Wasserstoff, $C_1$-$C_4$-Alkyl oder $C_2$-$C_3$-Hydroxyalkyl ist,

B° $-(CH_2)_m-$ oder eine der folgenden Gruppen bedeutet:

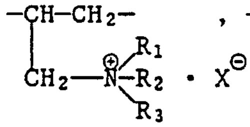

,

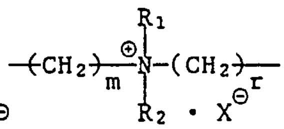

oder

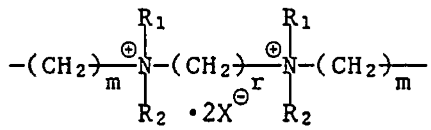

,

wobei m und r unabhängig voneinander 2 oder 3 bedeuten,

$R_{33}$ eine Bedeutung von $R_3$ hat oder für eine Gruppe der Formeln Id und Ie

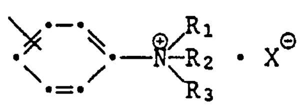

(Id)

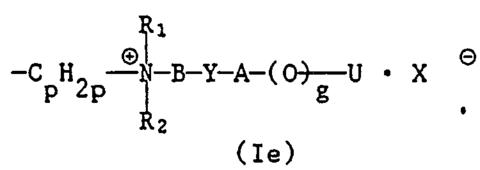

(Ie)

steht, worin g, p, B, Y, A und U die zuvor genannte Bedeutung haben, $R_1$, $R_2$ und $R_3$ unabhängig voneinander Wasserstoff, $C_1$-$C_8$-Alkyl, durch ein -COOR″ oder 1 bis 3 OH substituiertes $C_1$-$C_8$-Alkyl, durch ein oder mehrere -O- unterbrochenes $C_2$-$C_8$-Hydroxyalkyl, mit einem OH substituiertes -($C_1$-$C_8$)Alkylen-$COO^{\ominus}$, -($C_1$-$C_8$)Alkyliden-$COO^{\ominus}$, -($C_2$-$C_8$)Alkylen-$SO_3^{\ominus}$ oder -($C_2$-$C_8$)Alkyliden-$SO_3^{\ominus}$, $C_3$-$C_5$-Alkenyl, $C_5$-$C_7$-Cycloalkyl, Phenyl, Tolyl, Benzyl oder Glycidyl bedeuten, oder $R_1$ zusammen mit $R_2$ und gegebenenfalls mit $R_3$ sowie zusammen mit dem $\overset{\oplus}{N}$-Atom, an das sie gebunden sind, einen N-Heterocyklus bilden, der gegebenenfalls 1-3 N-Atome oder ein O-Atom als Ringglieder enthält,

R″ Wasserstoff oder $C_1$-$C_4$-Alkyl ist, und

$X^{\ominus}$, falls nicht in $R_1$, $R_2$, $R_3$ oder $R_{33}$ vorhanden, ein farbloses organisches oder anorganisches Anion bedeutet.

2. Aufzeichnungsmaterial gemäss Anspruch 1 von Verbindungen der Formel I, worin in der Formel Ia g = 0 ist.

3. Aufzeichnungsmaterial gemäss Anspruch 2, worin $R_{33}$ in der Formel Ia eine Bedeutung von $R_3$ hat.

4. Aufzeichnungsmaterial gemäss Anspruch 3, worin die Formel Ia einer Gruppe der Formel If

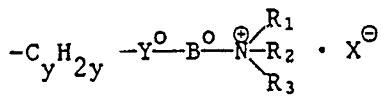 (If)

Y° -COO-, CONH- oder

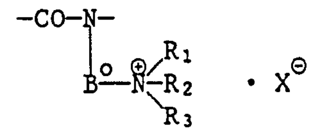

bedeutet, y eine Zahl von 0 bis 6 ist, und p, B°, $R_1$, $R_2$ und $R_3$ die in Anspruch I genannte Bedeutung haben.

5. Aufzeichnungsmaterial gemäss Anspruch 1, worin die Verbindung der Formel I einer Verbindung der Formel II oder III

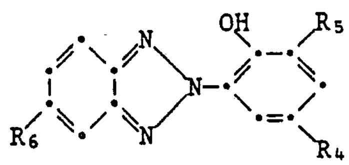

(II)

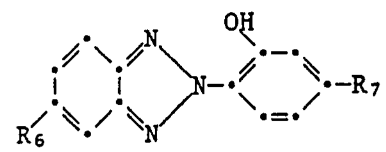

(III)

entspricht, worin

$R_4$ Halogen, $C_1$-$C_4$-Alkoxy, $C_1$-$C_8$-Alkyl, $C_5$-$C_6$-Cycloalkyl, Phenyl, Phenyl-$C_1$-$C_4$-Alkyl oder eine Gruppe der Formel Ia ist,

$R_5$ und $R_6$ unabhängig voneinander Wasserstoff sind, oder eine Bedeutung von $R_4$ haben,

$R_7$ OH, $C_1$-$C_8$-Alkoxy, welches durch 1 bis 3 OH oder 1 bis 2 -COOH substituiert sein kann, $C_1$-$C_8$-Alkanoyloxy, eine Gruppe der Formel Ia mit g = 1 oder Glycidyloxy ist,

unter der Bedingung, dass mindestens eines von $R_4$, $R_5$ und $R_6$ in der Formel II und mindestens eines von $R_6$ und $R_7$ in der Formel III für eine Gruppe der Formel Ia steht.

6. Aufzeichnungsmaterial gemäss Anspruch 5, worin $R_4$ Halogen, $C_1$-$C_5$-Alkyl, Cyclohexyl oder eine Gruppe der Formel Ia ist, $R_5$ Wasserstoff ist oder eine Bedeutung von $R_4$ hat, $R_6$ für Wasserstoff, Halogen oder eine Gruppe der Formel Ia steht, und $R_7$ OH, gegebenenfalls mit 1 bis 3 OH substituiertes $C_1$-$C_4$-Alkoxy, Glycidyloxy, $C_2$-$C_3$-Alkanoyloxy oder eine Gruppe der Formel Ia mit g = 1 bedeutet.

7. Aufzeichnungsmaterial gemäss Anspruch 6, worin in der Formel II $R_4$ $C_1$-$C_4$-Alkyl oder eine Gruppe der Formel If bedeutet, $R_5$ Wasserstoff, $C_1$-$C_4$-Alkyl oder eine Gruppe If ist, und $R_6$ Wasserstoff, Cl oder eine Gruppe If bedeutet, worin y für 0 steht, unter der Bedingung, dass mindestens eines von $R_4$, $R_5$ und $R_6$ eine Gruppe der Formel If darstellt.

8. Aufzeichnungsmaterial gemäss Anspruch 1, worin die Verbindung der Formel I einer Verbindung der Formel V

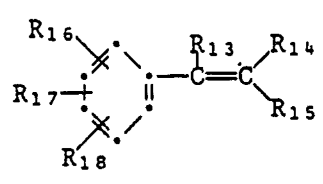 (V)

entspricht, worin

$R_{13}$ Wasserstoff, $C_1$-$C_8$-Alkyl, $C_3$-$C_8$-Alkenyl, Phenyl-$C_1$-$C_4$-Alkyl oder eine Gruppe der Formel

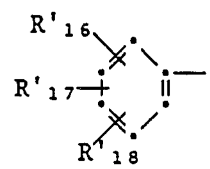

bedeutet, $R_{14}$ und $R_{15}$ unabhängig voneinander C≡N, -CO-$C_1$-$C_4$-Alkyl, -COO-$R_{19}$ oder -CO-$NR_{20}R_{21}$ bedeuten, $R_{16}$, $R_{17}$, $R_{18}$ $R'_{16}$, $R'_{17}$ und $R'_{18}$ unabhängig voneinander für Wasserstoff, $C_1$-$C_4$-Alkyl, Phenyl, Glycidyl, OH, Halogen, gegebenenfalls mit 1 bis 3 OH substituiertes $C_1$-$C_4$-Alkoxy, Phenoxy, Benzyloxy oder eine Gruppe der Formel Ia stehen, und $R_{19}$, $R_{20}$ oder $R_{21}$ unabhängig voneinander Wasserstoff, $C_1$-$C_4$-Alkyl, welches gegebenenfalls mit 1 bis 3 OH substituiert ist, $C_5$-$C_7$-Cycloalkyl, Phenyl, Benzyl, Tolyl, Glycidyl oder eine Gruppe der Formel Ig

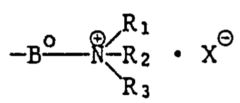

(Ig)

bedeuten, worin B°, $R_1$, $R_2$ und $R_3$ die in Anspruch 1 genannte Bedeutung haben,
unter der Bedingung, dass mindestens eines von $R_{16}$, $R_{17}$, $R_{18}$, $R'_{16}$, $R'_{17}$ oder $R'_{18}$ eine Gruppe der Formel Ia oder mindestens eines von $R_{19}$, $R_{20}$ oder $R_{21}$ eine Gruppe der Formel Ig bedeuten.

9. Aufzeichnungsmaterial gemäss Anspruch 8, worin die Verbindung der Formel V einer Verbindung der Formel Va

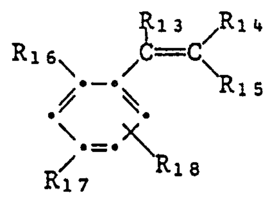

(Va)

entspricht, worin
$R_{13}$ Wasserstoff, $C_1$-$C_4$-Alkyl oder eine Gruppe

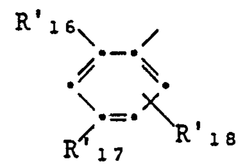

darstellt, und $R_{14}$, $R_{15}$, $R_{16}$, $R_{17}$, $R_{18}$, $R'_{16}$, $R'_{17}$ und $R'_{18}$ die in Anspruch 10 genannte Bedeutung haben.

10. Aufzeichnungsmaterial gemäss Anspruch 1, worin die Verbindung der Formel I einer Verbindung der Formel VI

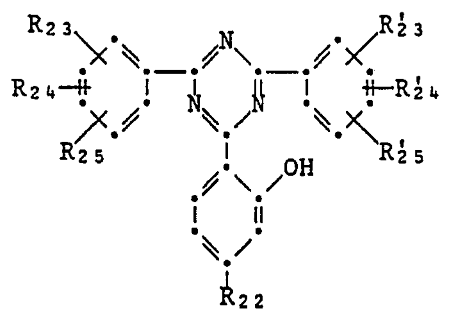

(VI)

entspricht, worin $R_{22}$ eine Gruppe der Formel Ia mit g = 1 ist und $R_{23}$, $R_{24}$, $R_{25}$, $R'_{23}$, $R'_{24}$ und $R'_{25}$ für Wasserstoff, OH, Halogen, $C_1$-$C_4$-Alkoxy, $C_1$-$C_4$-Alkyl, Phenyl oder eine Gruppe der Formel Ia mit g = 1 stehen.

11. Aufzeichnungsmaterial gemäss Anspruch 10, worin die Verbindung der Formel VI einer Verbindung der Formel VIa

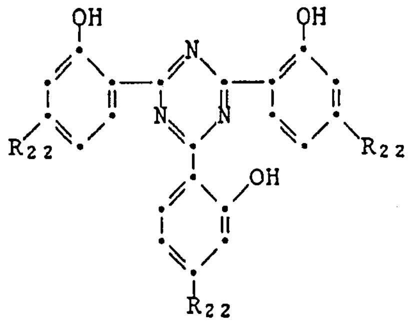

(VIa)

entspricht, und $R_{22}$ für eine Gruppe der Formel Ia mit g = 1 steht.

12. Aufzeichnungsmaterial gemäss Anspruch 1, worin $X^{\ominus}$ in der Formel Ia ist Chlorid oder Bromid, $C_1$-$C_4$-Alkyl-$COO^{\ominus}$, $C_1$-$C_4$-Alkyl-$OSO_3^{\ominus}$ oder $R^o$-$SO_3^{\ominus}$ bedeutet, wobei $R^o$ für Methyl, Tolyl oder -$CF_3$ steht.

13. Aufzeichnungsmaterial gemäss Anspruch 1, dadurch gekennzeichnet, dass das Aufzeichnungsmaterial für Tintenstrahldruck geeignet ist.

14. Aufzeichnungsmaterial gemäss Anspruch 13, dadurch gekennzeichnet, dass es aus einem Trägermaterial mit einer durch Tintenstrahl bedruckbaren Oberfläche besteht.

15. Aufzeichnungsmaterial gemäss Anspruch 14, dadurch gekennzeichnet, dass es ein beschichtetes Papier ist.

16. Verfahren zur Herstellung eines gegen Lichtschädigung stabilisierten Aufzeichnungsmaterials für den Tintenstrahldruck, dadurch gekennzeichnet, dass man einen zweidimensionalen Träger mit einer Beschichtungsmasse beschichtet, welche mindestens eine Verbindung der Formel I gemäss Anspruch 1 enthält.

17. Verwendung von Verbindungen der Formel I gemäss Anspruch 1 als Lichtschutzmittel für Aufzeichnungsmaterialien.

18. Tinte enthaltend als Stabilisator mindestens eine Verbindung der Formel I

$U(SOL)_n$ (I),

worin n eine Zahl von 1 bis 4 bedeutet,

U ein Rest eines UV-Absorbers vom Typ Hydroxyphenyl-benztriazol, Zimtsäure oder Hydroxyphenyl-Triazin ist,

SOL, gleich oder verschieden, eine Gruppe der Formel Ia

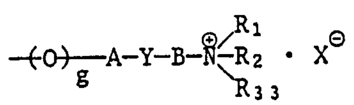

(Ia)

bedeutet, worin

g für 0 oder 1 steht,

A eine direkte Bindung, $C_1$-$C_6$-Alkylen oder -Alkyliden, oder eine Gruppe der Formel Ib

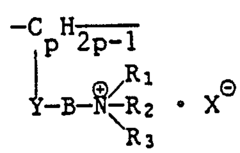

(Ib)

bedeutet, wobei p eine Zahl von 1 bis 6 ist,

Y eine direkte Bindung oder eine der folgenden Gruppen ist:

-CO-, -COO-, -OOC-, -CO-N(R′)-, -(R′)N-CO-, -SO₂-N(R′)-, -(R′)N-SO₂-,

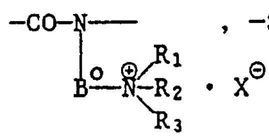 , 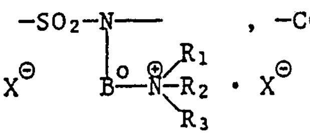 , 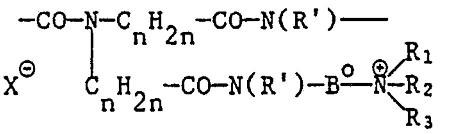 oder

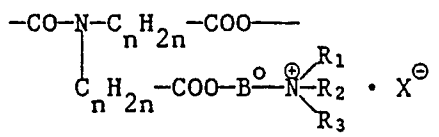 ,

B eine direkte Bindung bedeutet oder ein gegebenenfalls mit OH substituiertes $C_2$-$C_6$-Alkylen oder -Alkyliden ist, welches gegebenenfalls durch ein -O- oder durch ein oder zwei -$\overset{\oplus}{N}X^{\ominus}(R')_2$- unterbrochen ist, oder eine Gruppe der Formel Ic

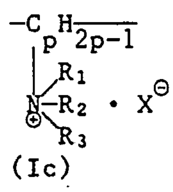 oder 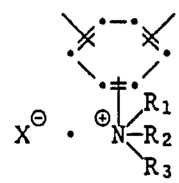

(Ic) (IC)

ist, oder die Gruppe

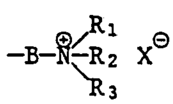

für einen gesättigten oder ungesättigten 1 bis 3-kernigen N-Heterocyklus steht, der 1-4 N-Atome als Ringglieder enthält, von denen mindestens eines quaterniert ist, unter den Bedingungen, dass
a) im Falle g = 1, A, Y und B nicht gleichzeitig eine direkte Bindung bedeuten;
b) im Falle g = 1 und A = direkte Bindung, Y -CO- oder eine direkte Bindung bedeutet; und
c) im Falle B = eine direkte Bindung, auch Y eine direkte Bindung ist;
R′ Wasserstoff, $C_1$-$C_4$-Alkyl oder $C_2$-$C_3$-Hydroxyalkyl ist,
B° $-(CH_2)_m-$ oder eine der folgenden Gruppen bedeutet:

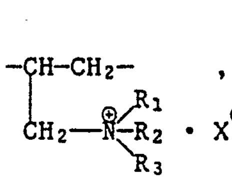 , 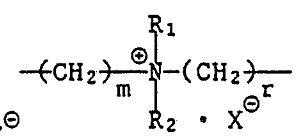 oder 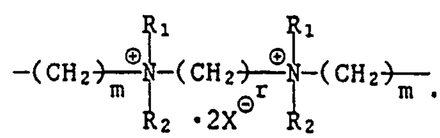 ,

wobei m und r unabhängig voneinander 2 oder 3 bedeuten,
$R_{33}$ eine Bedeutung von $R_3$ hat oder für eine Gruppe der Formeln Id und Ie

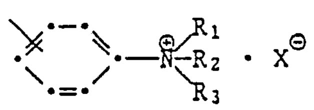

(Id)

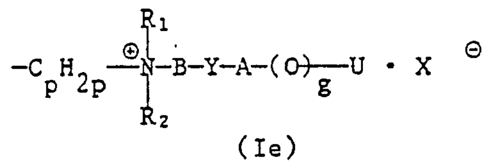

(Ie)

steht, worin g, p, B, Y, A und U die zuvor genannte Bedeutung haben, $R_1$, $R_2$ und $R_3$ unabhängig voneinander $C_1$-$C_8$-Alkyl, durch ein -COOR″ oder 1 bis 3 OH substituiertes $C_1$-$C_8$-Alkyl, durch ein oder mehrere -O- unterbrochenes $C_2$-$C_8$-Hydroxyalkyl, mit einem OH substituiertes -($C_1$-$C_8$)-Alkylen-$COO^{\ominus}$, -($C_1$-$C_8$)Alkyliden-$COO^{\ominus}$, -($C_2$-$C_8$)Alkylen-$SO_3^{\ominus}$ oder -($C_2$-$C_8$)Alkyliden-$SO_3^{\ominus}$ , $C_3$-$C_5$-Alkenyl, $C_5$-$C_7$-Cycloalkyl, Phenyl, Tolyl, Benzyl oder Glycidyl bedeuten, oder $R_1$ zusammen mit $R_2$ und gegebenenfalls mit $R_3$ sowie zusammen mit dem $\overset{\oplus}{N}$-Atom, an das sie gebunden sind, einen N-Heterocyklus bilden, der gegebenenfalls 1-3 N-Atome oder ein O-Atom als Ringglieder enthält, R″ Wasserstoff oder $C_1$-$C_4$-Alkyl ist, und $X^{\ominus}$, falls nicht in $R_1$, $R_2$, $R_3$ oder $R_{33}$ vorhanden, ein farbloses organisches oder anorganisches Anion bedeutet.

19. Tinte gemäss Anspruch 18, dadurch gekennzeichnet, dass die Tinte für den Tintenstrahldruck verwendet wird.

20. Tinte gemäss Anspruch 18, dadurch gekennzeichnet, dass die Tinte Wasser enthält.

21. Tinte gemäss Anspruch 18, dadurch gekennzeichnet, dass diese den Stabilisator der Formel I in einer Konzentration von 0,01 bis 20 Gew.-%, insbesondere von 0,5 bis 10 Gew.-%, enthält.

22. Tinte gemäss Anspruch 21, dadurch gekennzeichnet, dass diese zusätzlich mindestens einen wasserlöslichen Azofarbstoff enthält.

23. Verwendung von Verbindungen der Formel I gemäss Anspruch 18 als Stabilisator für organische Materialien.

24. Verwendung gemäss Anspruch 23 als Stabilisator für Aufzeichnungsmaterialien für den Tintenstrahldruck.

25. Verwendung gemäss Anspruch 23 als Stabilisator für Tinten für den Tintenstrahldruck.

26. Zusammensetzung enthaltend ein organisches Material und mindestens eine Verbindung der Formel I gemäss Anspruch 18 als Stabilisator.

27. Zusammensetzung gemäss Anspruch 26, dadurch gekennzeichnet, dass das organische Material ein synthetisches Polymer ist.

28. Zusammensetzung gemäss Anspruch 26, dadurch gekennzeichnet, dass das organische Material ein Lack ist.